# EUROPEAN PATENT SPECIFICATION

(11) **EP 3 021 752 B1**
(45) Date of publication and mention of the grant of the patent: **04.03.2020**
(21) Application number: 14826526.7
(22) Date of filing: 17.07.2014
(51) Int. Cl.: A61B 5/15, A61M 5/42, A61M 5/44, A61B 5/00, A61B 5/151, A61B 5/157, A61B 5/145

(54) **BLOOD SAMPLING DEVICE**
BLUTPROBENAHMEVORRICHTUNG
DISPOSITIF DE PRÉLÈVEMENT D'ÉCHANTILLONS DE SANG

(30) Priority: 17.07.2013 US 201361847094 P
(43) Date of publication of application: 25.05.2016
(73) Proprietor: Glucocheck Ltd., 52522 Ramat Gan (IL)
(72) Inventor: EREZ, Uri, 7570011 Rishon Lezion (IL)
(74) Representative: AWA Denmark A/S
(86) International application number: PCT/IL2014/050648
(87) International publication number: WO 2015/008288

(56) References cited:
- WO-A1-2010/109461
- FR-A1- 2 795 629
- US-A- 5 578 014
- US-A1- 2002 082 522
- US-A1- 2006 217 636

## Description

### FIELD

This invention relates to medical devices and methods for sampling blood, and more specifically to medical devices and methods for monitoring blood parameters/values.

### BACKGROUND

US 2006/0217636 A1 discloses a lance apparatus suited for forming an opening in the skin of a person to make blood available for medical testing, the device optionally including thermoelectric elements operable to heat and/or cool the skin, where the skin should be heated/cooled to a temperature configured to reduce the pain associated with the piercing, and the heating/cooling may be used to adjust the temperature to a comfortable level.

FR 2795629 A1 discloses a device for delivering an anesthetic, including thermoelectric means for cooling/heating the skin. The device serves to reduce the pain of the initial phase where the needle goes through the skin. FR 2795629 A1 further teaches that preferably cooling is used, but that in certain cases, such as in the case of abscess, heating may also be used. The cooling/heating is however exclusively applied before the needle penetrating the skin.

**Diabetes mellitus** is a group of metabolic diseases characterized by high blood glucose (blood sugar) either because insulin production is inadequate, or because body's cells do not respond properly to insulin, or both. The two major types of diabetes are:
**Diabetes Type I** - is characterized by a complete lack of insulin production. Diabetes type I is referred to as insulin-dependent diabetes, juvenile diabetes, or early-onset diabetes. Type I diabetes may appear at relative young age, before 40, often in early adulthood or teenage years. Type I diabetes is nowhere near as common as type II diabetes, approximately 10% of all diabetes cases are type I. Patients with type I diabetes will need to inject insulin for the rest of their life. All insulin dependents must ensure proper blood-glucose levels by carrying out regular blood tests and following a special diet. Between 2001 and 2009, the prevalence of type I diabetes among the under 20s in the USA rose 23%, according to *SEARCH for Diabetes in Youth* data issued by the CDC (Centers for Disease Control and Prevention).
**Diabetes Type II** - is characterized by insufficient insulin production or insulin resistance due to failure of body cells to react to insulin. Approximately 90% of all diabetes cases worldwide are type II. Type II diabetes symptoms may be controlled by losing weight, following a healthy diet, doing plenty of exercise, and monitoring blood glucose levels. However, type II diabetes is typically a progressive disease and patients may need to take insulin usually in tablet form.

Researchers from Mayo Clinic Arizona showed that gastric bypass surgery can reverse type II diabetes and that within three to five years the disease may recure in approximately 21% of the patients. Yessica Ramos, MD., noted that "The recurrence rate was mainly influenced by a longstanding history of Type II diabetes before the surgery. This suggests that early surgical intervention in the obese, diabetic population will improve the durability of remission of Type II diabetes."

Patients with type I diabetes are treated with regular insulin injections, as well as a special diet and exercise. Patients with Type II diabetes are usually treated with tablets, exercise and a special diet, but sometimes insulin injections are also required. If diabetes is not adequately controlled patients have a significantly higher risk of developing serious complications.

**Hypoglycemia,** also called insulin reaction, may occur due to a too low blood glucose. Hypoglycemia may occur also in treated diabetes pateints, and it can and must be detected and treated immediately. Regular meals and snacks are vital for keeping blood glucose levels as stable as possible and prevent Hypoglycemia. Hypoglycemia affects all the organs in the body including the brain. Hypoglycemia, if not treated quickly may deteriorate rapidly and patients may pass out. A patient who passes out due to hypoglycemia needs immediate treatment by a glucagon injection and may need emergency care treatment at a hospital.

Checking blood glucose regularly reduces the chances of experiencing hypoglycemia. The American Diabetes Association (ADA) recommends that if a diabetes patient feel a hypoglycemic reaction but cannot check his blood glucose, he better treat the reaction immediately and not wait untill he can check his blood glucose. The American Diabetes Association (ADA) recommends that after checking blood glucose and treateing hypoglycemia accordingly, the patient should wait 15 to 20 minutes and re-check his blood glucose. If the blood glucose is still low, it is recommended to repeat the treatment, e.g. eat more glucose containing food, to wait about 15-20 minutes and re-check blood glucose.

**Hyperglycemia** - may occur due to a too high blood glucose. Hyperglycemia needs to be detected and treated immediately since it is the major cause of serious complications related to diabetes. Hyperglycemia may occur due to a lack of insulin in the blood or to a type II diabetes insulin resistance. The main reason for hyperglycemia for a diabetes type I patient is that he or she has not taken enough insulin. For a type II diabetic patient, it could be the same reason, but also may occur due to insulin resistance. For a diabetes patient, overeating can bring on hyperglycemia, as can too little exercise on a given day. Mental stress can also generate Hyperglycemia.

Thus, diabetes patient blood glucose management is crucial and patients need to monitor and regulate their blood glucose in order to prevent occurrence of Hyperglycemia and Hypoglycemia.

**Self-Blood Glucose Monitoring (SBGM)** - is the key tool for managing diabetes disease enabling patients to achieve better control of their blood glucose. Accuracy of SBGM values "in real-time" is crucial especially for insulin injecting patients that calculate their insulin dosages according to blood glucose results in order to define the appropriate dosages and diet and also for type II noninsulin diabetics.

Balancing blood glucose levels allows preventing serious complications related to diebetes, such as damage to large blood vessels of the heart, brain, eyes and legs and also to meidum and small blood vessels (capillaries) of eyes, kidneys, feet and nerves. Other parts of the body may also be affected by diabetes, including the digestive system, genitalia, teeth and gums and the immune system. Accuracy of blood glucose values "in real-time" is highly needed especially for insulin injecting patients who have to calculate their insulin dosages, including other medications, and to define the appropriate diet according to blood glucose results.

Unfortuately, insulin dependent patients are requiered to conduct SBGM 6-8 times on daily basis. They are further required to monitor the effect of diabetes medications on blood sugar level and consecutively adjust insulin therapy, define the dosage and the diet, evaluate the effect thereof and of physical exercise on blood sugar levels, and identify blood sugar levels that are dangerously high or low. Type I diabetic patients are requiered to conduct SBGM at least once a day and preferrably 6-8 times per day.

Diabetics who suffer from hyperglycemia episodes need to be rapidly diagnosed/detected and managed, since prolonged hyperglycemia leads to dehydration, metabolic disturbances, and serious long-term cardiovascular complications. The American Diabetes Association (ADA) recommends SBGM for diabetes patients as a key component of their disease management program. Glycemic control is being recognized as a priority in the treatment of critically ill patients.

Blood glucose control reduces the incidence of diabetes-related complications, for example, stroke, heart attack, retinal hemorrhage, and amputation. A 10% improvement in glucose control reduces incidence of complications by 37%. Today, patients are seen episodically by caregivers and are expected to comply with complicated regimens of testing and treatment.

### Advantages of Fingertip SBGM from Medically Perspective

Fingertips SBGM is recognized by the FDA and medical staffs as standard of care for SBGM due to the following major physiological advantages of fingertips over alternate sites, such as the arm, thigh, or palm hand:
a) Fingetips provide high volume of blood samples due to the huge number of capillaries located in the dermis layer of the fingertip's skin at high density and to the thin fingertip skin.
b) Fingetips provide accurate blood glucose results due to the minimal lag time of blood glucose carried efficiently by the huge number of capillaries to the fingertip's skin layer from larger blood vessels.
c) Fingertips SBGM allows detecting hypo or hyper glycemia in real-time with no lag time which allows to provide immediate treating.

It should be noted that diabetics patient may suffer from blocked blood vessels resulting in poor blood flow which may further prevent obtaining sufficiet volume of blood samples for SBGM.

**Disadvantages of Fingertip SBGM from End Users Perspective** Unfortunately, fingertips SBGM suffer from two major drawbacks, which cause a major noncompliance problem for most diabetes patients:
(a) Physical pain - due to the high sensitivity of the fingertips' skin that contains a high density of pain receptors, the SBGM device's lancet injures fingertips pain receptors. Finger piercing, therefore, is followed by sharp and provoking physical pain. The pain is felt not only during skin piercing but lasts few days until the injury heals. Since fingertips pricking is required to be preformed daily, it leads to sore, callused, dysfunction and loss of sense of touch. The physical fingertip pricking pain, including the fear of pain, leads to a major noncompliance problem with fingertip SBGM.
(b) Sensitivity to cooling - fingertip skin contains many other sensory endings such as thermal fibers, which are very sensitive to cooling and heating, thus preventing a cooling anesthetic step that may cause cold-burning-pain that may be more painful than fingertip pricking.

According to Lutz Heinemann: "pricking the fingertip several time per day for many years/decades, is not only annoying to the patient but also has certain consequences: (1) development of massive scarring/callous formation and (2) loss of sensibility perception, hindrance. The pain associated with fingertips pricking is most probably the main reason (besides the costs) why patients refrain from SMBG. The pain might also induce a negative perception against diabetes and its therapy in general", [Finger Pricking Pain, a Never Ending Story, by Lutz Heinemann Ph.D, Journal of Diabetes Science & Technology, 2008, Sep; 2(5): 919-921].

Hawthorn et. al contemplate that "the pain associated with fingertips pricking is most probably the main reason why many patients refrain from SMBG. In turn, such reduced measurement frequency has a negative impact on metabolic control. The pain associated by fingertip prick induces a negative perception against diabetes and its therapy. From my point of view, much more attention should be given to the pain associated with fingertip pricking" [Sue Cradock, Jan Hawthorn, Journal of Diabetes Nursing, Nov.-Dec., 2002].

American Diabetes Association survey has found that "21% of Type I insulin users never checked their blood glucose", "Of those who are insulin dependents diabetes Type II - 47% never checked their blood glucose", "Among those with Type II diabetes who are not using insulin - 76% never checked blood glucose", 'Out of 23 million diabetic patients in the US only 1% test blood glucose regularly".

The statistical data disclosed hereinabove highlights the huge noncompliance problem with fingertip SBGM, which may be solved by a noninvasive glucometer or by replacing fingertip SBGM with alternate site SBGM.

**Advantages of Alternate site SBGM from End Users Perspective** - Alternate sites' skin is much less sensitive to pain caused by small injuries with a needle compared to fingertips' skin, due to smaller density of pain receptors at alternate site skin comparing to fingertips. Alternate site's skin is not so sensitive to thermal contact because it is poor with thermal fibbers, which allows a cooling step to be used as an anesthetic step, comparing to fingertips' skin which contains many thermal fibbers which avoids using a cooling step as an anesthetic step. in SBGM. It should be emphasized that precondition of using a cooling step as an anesthetic step in practical application by SBGM requires to replace fingertip SBGM for fingertip's-free alternate site SBGM. Thus it requires a BGM that meets the medical and FDA requirements for fingertip-free SBGM, i.e., producig large volume and sufficient volume of blood samples from an alternate site similar to fingertips and equalize the accuracy of blood glucose results of an alternate site to be equivalent to fingertips, which requires both increasing the power and velocity of blood flow in capillaries located close to the puncturig site/s.

### Disadvantages of alternate sites SBGM from Medically and Regulatory Perspective

- Unfortunately, alternate site skin SBGM suffer from two major physiological drawbacks, which prevents replacing fingertip SBGM by alternate site SBGM:
   (a) Insufficient volume of blood samples - non fingertips alternate sites' skin is significantly thicker than figertip's skin in healthy people, while many diabetic patients who suffer from a thicker skin in general includig the optional alternate sites. Thicker skin results in capillaries located deeper below the skin surface, while the blood flow in capillaries is too weak to push the viscous blood out of such a thick skin, which prevents providing a sufficient volume of blood sample from the alternate site. Additionally, diabetic patients suffer from blocked blood vessels, resulting in a lower blood flow, which also prevents providing sufficient volume of blood samples from alternate sites. Since BGM companies provide very thin/fine blood sampling lancets to reduce the pircing pain, the blood samples are insufficient.
   (b) Time lag in blood glucose concentration - the delay in equilibration of blood glucose testing in alternate sites, such as the arm or thigh, appears to be about 60 to 90 minutes after a meal or glucose load and up to 240 minutes following a combination of glucose load and insulin injection. Thus, the typical long time lag of blood glucose monoitoring in alternate site SBGM does not allow detection of hypoglycemia or hyperglycemia in real time when glucose concentration is changing.

The physiological disadvantageous of alternate site's skin from medically perspective and regulatory perspective described above, have led to serious concerns about the ability of to identify hypoglycemia using SBGM in alternate sites [Philip E. Knapp et al., DIABETES TECHNOLOGY & THERAPEUTICS, Volume 11, Number 4, 2009]. Additionally, according to Dr. Andreas Stuhr, medical officer of Roche Diabetes Care Division: "While alternative testing sites are less sensitive to pain, getting a big enough blood sample from them can be hard to do without some guidance, which may be why few people use alternative sites." "They may struggle trying to get enough blood from alternate sites. They waste a lot of costly test strips to get enough blood from alternative sites, and finally give up" [Andreas Stuhr, Cornel Hospital, NY City, Diabetes Self-Management, Alternate Site Testing].

**Efforts for replacing fingertips SBGM with alternate site SBGM** - alternate site SBGM is currently recommended by medical staffs and is approved by the FDA only as a second option to fingertips SBGM due to the disadvantageous described hereinabove.

Thus, there is a longfelt and unmet need to improve the accuracy of glucose measurements and increase blood sample volumes obtainable from alternate sites. There have been several attempts to increase blood samples volume from alternate sites by creating vacuum at the puncture site, heating or exerting pressure on the area surrounding the puncture site.

US Patent No. 6,605,048 discloses a vacuum device configured to draw blood samples from alternative puncture sites such as the forearm, abdomen or inner thigh and which may advantageously be used by diabetics. The device includes a hollow body which slidably receives a hollow plunger. A transparent cylindrical tip is attached to the lower end of the hollow body. A spring is carried inside the hollow body and hollow plunger, the lower end of the spring seating against a webbing formed in the transparent tip and the upper end seating against the upper end of the plunger. The device is actuatable by a thumb and two fingers of one hand. The plunger is depressed, the device is placed over a preexisting puncture site, and the plunger is released wherein the internal spring drives the plunger upwardly and creates a vacuum at the puncture site, allowing the user to easily see the formation of a droplet of adequate size for obtaining the necessary blood reading.

International Patent Application No. WO 1999/027852 discloses a method and an apparatus for obtaining a sample of interstitial fluid from a patient for subsequent diagnostic tests, e.g. glucose monitoring. The method comprises the steps of: (a) treating an area of the skin with vacuum or heat or both vacuum and heat to increase the availability of interstitial fluid at that area of the skin; (b) forming an opening in the treated area of the skin; and (c) extracting the sample of interstitial fluid from the opening in the skin, with the aid of vacuum and stretching of the skin. The apparatus for carrying out the described method comprises: (a) a device for forming an unobstructed opening in an area of skin from which the sample is to be extracted, preferably a lancing assembly; and (b) a vacuum pump.

CA Patent No. 2,476,308 is directed to analyte monitoring/drug (pharmaceutical agent) delivery device. CA Patent No. 2,476,308 also encompasses various techniques for enhanced blood collection. Figures 20a and 20b show the use of heating sources (e.g., infrared emitting LED) to heat the tissue near the collection site. Heating the capillary structure of the tissue at the collection site may improve the blood supply and enhance collection of blood using micro-needles.

US Patent Application No. 2009/0177224 discloses methods and apparatus for blood sampling from skin capillaries. Blood extraction is improved by applying gradient pressure in a proximal to distal direction of an extremity. Blood is extracted and sampled from the skin capillaries with a blood sampling mechanism of at least a lancet and a testing kit.

US Patent No. 6,491,709 discloses a new tip for a conventional lancer that provides improved blood flow from a lancet puncture site not located on a patient's finger. The new lancer tip includes a plurality of crenellations that exert rotational force on the skin surrounding the lancet puncture site when the lancer is rotated to enhance blood flow from the lancet puncture site.

However, the methods described hereinabove configured to increase or decrease pressure on and around the puncture site inevitably increase pain and injury associated with the blood sampling.

In recent years several devices for non-fingertips alternate sites blood sampling have been developed. Several attempts were done to increase the volume of blood samples from alternate sites by new BGM devices or improve the BGM results accuracy. For example, FreeStyle device manufactured by Abbott disclosed new blood glucose test strips, which require smaller volume of blood samples. Additional attempts to increase blood samples volume were based on creating vacuum at the puncture site. Lancing devices including replaceable plastic part at the front part thereof were produced, wherein the plastic part were configured to create suction action, when applied to the puncture site and pressed several times against it. However, the rigid material of the plastic part may not allow effectively creating vacuum at the puncture site and large blood samples may not be obtained.

The FDA has recently approved new BGMs for alternate site SBGM including disposable blood glucose test strips that require smaller volume of blood samples, and lancing devices including a replaceable part for creating vacuum, such as Ascensia Breeze, Elite, Dex, Dex2, Bayer/Ascensia, FreeStyle and TheraSense, approved for fingertip, upper arm, thigh, calf and fleshy parts of the hand; Precision Sof- J. UltraSmart, InDuo LifeScan approved for fingertips, forearm, In-Duo: Ultra meter with insulin delivery device, Active, Compact Roche Diagnostics approved for fingertip, palm, forearm, upper arm, thigh or calf.

Unfortunately, these BGM devices are approved by the FDA for testing of alternate sites not as a replacement of fingertips SBGM, but in addition to fingertip SBGM as a second option only, and not instead of figertips. The FDA approved some BGMs also for alternate sites suvbject to to significant limitations such as: only fingertip SBGM is allowed, while alternate sites for: diabetes type I who are insulin dependents (10% of all diabetics), for diabetics who suffer of prompt changes in blood glucose, after meals, after exercise, during illness, etc., where only fingertip SBGM is allowed. In fact such precondition restrictions turns using alternate sites as useless.

Dr. Andreas Stuhr further contemplates that "even with new meters that require smaller blood samples it is still trickier getting enough blood to check your glucose level from an alternative site".

In fact, the only alternate site that meets the requirements of the FDA and medical staffs from the perspective of accuracy of glucose results similar to fingertip, is the palm hand and/or the inner fleshy side of fingers. Medical studies have proved that the accuracy of glucose results in those sites are similar to sampling from fingertips, and therefore, the FDA also recognized those sites for SBGM to be equivalent to fingertips.

Palm hand includes more capillaries than other alternate sites, and therefore, the velocity of blood flow in capillaries located in the palm is more or less similar to fingertip. The FDA does not approve palm hand SBGM as a replacement for fingertip SBGM, but in addition to fingertip SBGM, as a second option, because the palm, such as other alternate sites, does not provide sufficient volume of blood samples even with new blood glucose meters, requiring smaller volume blood samples for analysis. It should be emphesized that alternate sites' skin of many diabetics is thicker comparing to healthy people skin where the capillaries are located at a deeper depth below the skin surface, thus additionally decreasing the volume of obtainable blood samples.

Another approach towards using the alternate sites for glucose testing is to non-invasively improve the measurement accuracy. US Patent No. 6,998,247 discloses methods for calibrating noninvasive or implantable glucose analyzers utilize either alternative invasive glucose determinations or noninvasive glucose determinations for calibrating noninvasive or implantable glucose analyzers. Use of an alternative invasive or noninvasive glucose determination in the calibration allows minimization of errors due to sampling methodology, and spatial and temporal variation that are built into the calibration model. An additional method uses statistical correlations between noninvasive and alternative invasive glucose determinations and traditional invasive glucose determinations to adjust noninvasive or alternative invasive glucose concentrations to traditional invasive glucose concentrations. The methods provide a means for calibrating on the basis of glucose determinations that reflect the matrix observed and the variable measured by the analyzer more closely. A glucose analyzer couples an invasive fingerstick meter to a noninvasive glucose analyzer for calibration, validation, adaptation, and safety check of the calibration model embodied in the noninvasive analyzer.

Alternative approach towards reducing the dependency of the glucose measurement on employing fingertips is measuring glucose from body fluids other than blood. MiniMed Continuous Glucose Monitoring System, approved by the FDA, includes an insulin pump with small plastic catheter that is inserted just under the skin, a sensor inserted into the subcutaneous tissue and a convention BGM. The sensor wirelessly transmits glucose results from the subcutaneous tissue every 5 minutes, while the BGM calculator for dosage translates it to dosage, and the insulin pump inject insulin accordingly. However, this product was not approved by the FDA as a replacement of fingertips SBGM, but in addition and as a second option, while the first option for SBGM remains fingertips SBGM, because it measures glucose not from the blood but rather from the subcutaneous tissue, resulting in inaccurate glucose results. Furthermore, this product is for use only for insulin dependents (type I), who are only 10% out of all diabetic patients, while the rest 90% of type II diabetic patients should test SBGM by current fingertips BGMs

Another device, approved by the FDA, is GucoWatch - a minimal-invasive, watch-like device, configured to measure glucose contents by tiny electronic currents. GucoWatch is configured to draw a small amount of fluid from the skin 3 times per hour for up to 12 hours. A drawback of GucoWatch device is a reduced accuracy due to measurement of glucose not directly from blood and thus it may only provide an indication for too high or too low glucose. Hence, GucoWatch is approved by the FDA not as a replacement to fingertips SBGM, but in addition to fingertips which remains the first option for SBGM.

**Cryo-local-anesthesia and SBGM** - Cryo-local-anesthesia is a well known method used to prevent pain. The method is based on cooling the dermis skin layer where pain receptors are located to temperatures of +8 degrees C or below (which may be defined as the critical level of local-anesthesia). At these temperatures, pain receptors are locked, pain information to the brain is blocked, resulting in cryo-local-anesthesia and no pain is felt. This method may be effective especially for athletics who suffer of dry blows injuries (since in such injuries there may be no need to detect when the temperature in the dermis skin layer drops to said critical levels of local-anesthesia). Cryo-local-anesthesia method may not effective when the temperatures in the dermis skin layer to be punctured for example with a needle are above +8 degrees C, becsuse at higher temperatures the pain receptors are not blocked and pain information to the brain is transmitted.

However, cryo-local-anesthesia techniques cannot be used with fingertips SBGM, since fingertips' skin contains high density of thermal fibers, which are sensitive to thermal contact. The sensitivity of the skin to thermal cotact prevents using a cooling step as an anesthetic step by fingertip SBGM (cooling fingertip's skin may be more painful than fingertip pricking).

Ethyl Chloride may be used for cryo-local-anesthesia. Ethyl Chloride is sprayed on injured sites at very low temperature of about -18 degrees C. Athlets, who suffer from dry blow injuries, use Ethyl Chloride, without measuring the temperature of the skin. However, Ethyl Cloride is not used in surgeries, because it will require meausring and maintaining skin temperature below +8 degrees.

Patents that disclose inducing cryo-local-anesthesia, such as Internationational Publication Number WO 2008/08144 A2, and International Publication Number WO 2010/109461 A1, use only a cooling step which reduces the temperarure of the skin without ensuring that the dermis skin is maintained below +8 degrees C.

SBGM with cryo-local-anesthesia method based on the function of said predetermied temperatures of the critical level of cryo-local-anesthesia, requires a blood lancing device capable of providing a cooling system and a temparture sensing and communication means. The temperature sensing means should measusre continually the internal dermis temperature in the part of the skin to be puctured, and when the temperaure drops to below +8 degrees, the skin pricking may be perfomed after receiving a communication from the temparture sensing means. However, using an invasive implanted temperature sensing means is difficult and is not applicable to blood sampling devices. Unfortunately, such an invasive implanted temperature sensing means does not meet the regulatory agencies requirements and therefore, cryo-local-anesthesia can not be implemented in practical solution by fingertip SBGM which is standard of care.

Hence, a precondition to use in practical application cryo-local-anesthesia method with SBGM requires: (1) a BGM which meets the medical and FDA requirements for a fingertip-free alternate site SBGM that its skin includes lower density of thermal fibers and may be cooled to +8 C or below. (2) patents for cryo-local-anesthesia based on cooling the skin as a fuction of said predetermied temperatures is impracticle, which require a different practical solution such as described herein below of the present invention.

Thus, there is a longfelt and unmet need for an altrnate site SBGM method that use cryo-local-anesthesia to prevent or to reduce pain, capable of increasing blood sample volume and of providing accurate blood glucose results equivalent to figertips which meet the medical and FDA requirements for a non-fingertip-free SBGM.

The foregoing examples of the related art and limitations related therewith are intended to be illustrative and not exclusive. Other limitations of the related art will become apparent to those of skill in the art upon a reading of the specification and a study of the figures.

### SUMMARY OF THE DISCLOSURE

The following examples and aspects thereof are described and illustrated in conjunction with systems, tools and methods which are meant to be exemplary and illustrative, not limiting in scope.

According to a first aspect, the blood sampling device of the present disclosure is configured to solve two major physiological drawbacks of non-fingertips alternate sites piercing from medically and regulatory perspective of SBGM, providing a SBGM device configured to meet the medical and FDA regulatory requirements, and to replace fingertips SBGM devices providing a new standard of care pain-free and fingertips-free.

According to some examples, the blood sampling device of the present disclosure provides a fingertip-free and pain-free SBGM device configured to produce sufficient blood sample volumes from non-fingertips alternate site/s similar to fingertips blood samples. The blood sampling device is configured to get from alternate site/s accurate blood glucose results equivalent to fingertips SBGM.

The blood sampling device is configured to cool the skin target area, thereby to create a blockage of blood flow in capillaries located close to the puncture site and to provide a cryo-local-anesthesia before and during piercing the non-fingertips alternate site.

According to some examples, the blood sampling device of the present disclosure is configured to increase the inner pressure and velocity of the blood flow in capillaries located close to the non-fingertip alternate site before piercing by cooling the skin area to be punctured and furthermore, to boost the blood flow ejected after piercing by heating the skin target area which may provide significant advantages from the perspective of alternate site SBGM. Diluting the viscous blood close to the puncture site to be more watery helps in producing large volume blood samples from alternate site's skin, it also increases the velocity of blood flow which avoids the lag time of reaching glucose concentration to the cells of alternate site's skin resulted in accuracy of blood glucose results from an alternate site equivalent to fingertips, and it allows supplying more oxygen to alternate site injuries resulted in faster healing of skin pricking injuries.

According to some examples, the blood sampling device includes: thermo-conductive member/s configured to cool and heat the skin target area and a piercing system, that may include: a piercing element configured to pierce the skin target area and a piercing mechanism for extending and retracting the skin piercing element.

According to some examples, the blood sampling device may be a stand-alone unit. According to other examples, the blood sampling device may be coupled/integrated with a blood test meter.

According to further examples, the blood test meter may be configured to be easily attached and removed from the blood sampling device. According to some examples, the blood test meter may be configured to measure blood glucose levels. According to other examples, the blood test meter may be configured to measure other blood parameters/values, such as, but not limited to, hemoglobin, ketones, cholesterol, blood coagulants or combinations thereof.

According to some examples, the blood sampling device is configured to cool the skin target area. According to further examples, the blood sampling device is configured to provide non-invasive cooling of the skin target area.

According to other examples, the blood sampling device may be configured to heat the skin target area. According to further examples, the blood sampling device may be configured to provide non-invasive heating of the skin target area. According to yet further examples, the blood sampling device is configured to provide cooling and subsequent heating of the skin target area. According to still further examples, the blood sampling device may be configured to provide non-invasive cooling and subsequent non-invasive heating of the skin target area.

According to some examples, the blood sampling device further includes an operating control unit configured to decrease the temperature of the thermo-conductive member and then to increase the temperature of the thermo-conductive member, wherein the operating control unit is further configured to trigger the extension of the piercing element.

According to some examples, the operating control unit may include a programmed microcontroller which may control and operate all systems of the blood sampling device.

According to additional examples, the operating control unit may be configured to trigger the extension of the piercing element upon receiving an indication with regard to the thermo-conductive member/s reaching a predetermined cooling time, a predetermined temperature or both.

According to further examples, the operating control unit may be configured to switch between decreasing the temperature of the thermo-conducting element and increasing the temperature of the thermo-conducting element upon the retraction of the piercing element or upon receiving an indication with regard to the thermo-conductive member reaching a predetermined cooling time, a predetermined temperature, or any combination thereof.

According to further examples, the operating control unit of the blood sampling device may communicate with the operating control unit of the integrated blood test meter or the BGM.

According to some examples, the thermo-conductive member/s may be configured to directly contact the skin target area, including a skin piercing point. According to further examples, the thermo-conductive member/s may be configured to directly contact the skin target area, including a skin piercing point during cooling and heating of the skin target area where pain receptors that may conduct pain are located and should preferably be blocked by a cooling anesthetic step not as a function of the predetermined temperature of the dermis skin layer to be punctured, but as a function of predetermined time needed by the present disclosure until no pain is felt in practical application. Cooling directly the pain receptors at the skin target area piercing point provides effective cryo-local-anesthesia that blocks the specific pain receptors to be injured during skin pricking, while cooling other parts of the skin is in effective.

According to additional examples, the thermo-conductive member/s may be configured to directly contact the skin target area, including a skin piercing point before, during and after piercing of the skin target area. According to still further examples, the thermo-conductive member/s may further be configured to exert some pressure on the skin target area. According to some examples, the exerted some pressure may improve the blockage of blood flow in capillaries located close to the piercing point and improve the thermo-conductive member/s to the skin target area contact, thereby to improve cooling and heating of the skin target area.

According to further examples, the thermo-conductive member/s may include a straight, convex, concave surface that may comprise a thin plate.

Without being limited by any specific theory or mechanism of action, pressing the thermo-conductive member/s against the skin target area may result in both creating an additional blockage of blood flow in capillaries, or creating a more effective blockage of blood flow in capillaries located below the blockage, which allows creating from second to second higher increased inner blood pressure within capillaries located close to the puncturing site.

According to further examples, providing cooling and heating steps allow blocking blood flow in capillaries and creating high blood pressure within the capillaries located below the blockage, and after pricking the skin, removing the blockage followed by releasing the accumulated high blood pressure. It is resulted in increasing the power and velocity of blood flow in capillaries located close to the puncturing site, producing sufficient volumes of blood samples and allows accuracy of blood glucose results obtained by non-fingertips alternate site SBGM to be similar to fingertips.

According to some examples, the blood sampling device may include at least one thermo-electric cooling (TEC) element in thermal contact with the thermo-conductive member/s, wherein the at least one TEC element may be configured to cool and/or heat the thermo-conductive member/s. According to some examples, the TEC element may include a cold end, configured to cool the thermo-conductive member/s and a hot end, configured to heat the thermo-conductive member/s. According to additional examples, the cold end and the hot end are interchangeable.

According to some examples, the device may include at least two TEC elements in contact with two movable-thermo-conductive members connected to two movable/retractable segments. According to further examples, the TEC elements may be movable together along with the movable-thermo-conductive members/segments. According to an exemplary example, the device may include four TEC elements. According to some examples, the thermoelectric cooling element may be powered by at least one electrical power source.

According to additional examples, the electrical power source may be a DC battery, external electric power supply, or a combination thereof.

According to some examples, the blood sampling device may include a heat absorber and/or an insulating partition connected to the TEC element, configured to absorb heat transferred from the hot end of the TEC element during cooling of the skin target area. The heat absorber may be the piercing element housing.

According to some examples, the heat absorber may include high thermal capacity materials in order to accumulate effectively heat from the hot end of the TEC element, or it may include cooling fins or pins that may be combined with a fan, and it may include artificial diamonds. Each possibility represents a separate example of the disclosure.

According to additional examples, the heat absorber may further include at least one TEC element, wherein the TEC element may be configured to contact the heat absorber with a cold end thereof, while the hot end of the TEC element is connected to additional heat absorber made of high thermal capacity material. Part of the heat absorber may be movable due to using retractable/movable segments connected to the TEC element. According to some embodiments, the heat absorber may include at least one temperature sensing means configured to communicate with an operating control unit microprocessor.

According to some examples, the thermo-conductive skin member/s-segments may be cooled to a temperature from about +10 to about -10 °C. According to further examples, the thermo-conductive member may be cooled to a temperature from about 10 °C to about -5 °C. According to other examples, the thermo-conductive member/s-segments may be cooled to a temperature from about +5 to about 0 °C.

According to additional examples, the thermo-conductive member/s-segments may be cooled to a temperature from about 0 to about -5 °C. According to further examples, the thermo-conductive member/s-segments may be cooled to a temperature from about -5 to about -10 °C. According to some examples, the skin movable thermo-conductive member/s-segments may be cooled to at least +10 °C. According to additional examples, the skin thermo-conductive member/s-segments may be cooled to not less than -10 °C.

According to further examples, the skin movable thermo-conductive member/s-segments may be cooled to a predetermined temperature/s at a predetermined time.

According to further examples, each thermo-conductive member/segment may include a TEC element and a heat absorber.

According to further examples, the skin movable-thermo-conductive members/segments may be heated to a temperature from about + 10 °C to about +50 °C.

According to still further examples, the skin movable thermo-conductive members/segments may be heated to not more than +50 °C. According to further examples, the skin movable-thermo-conductive members/segments may be heated to a predetermined temperature at a predetermined time.

According to alternative examples, the skin movable-thermo-conductive members/segments may be cooled by a vapor-compression refrigeration. According to further examples, the skin thermo-conductive member may be readily-removable from the blood sampling device. According to some examples, the thermo-conductive member/s-segments may be made of thermally-conductive materials. According to further examples, the skin thermo-conductive member may be removed from the blood sampling device and cooled externally.

According to some examples, the blood sampling device may include one or more temperature sensing means configured to communicate with the operating control unit.

According to some examples, the thermo-conductive member includes a heat absorber, configured to accumulate the excess of heat. According to some examples, the heat absorber may be in thermal contact with at least one temperature sensing means

According to some examples, the thermo-conductive member/s may be disposable. According to other examples, the thermo-conductive member/s may be covered by an interchangeable disposable cover/s. The interchangeable cover/s may comprise thin and flexible thermo-conductive materials such as, but not limited to, aluminum foil. According to some examples, the interchangeable cover/s may be sterilized.

According to some examples, the thermo-conductive member/s may be configured to provide an opening through which a protrusion of the piercing element may be facilitated.

According to further examples, the thermo-conductive member/s may include at least two segments.

According to some examples, the thermo-conductive member may include 2-10 segments, for example, 2 segments, 3 segments, 4 segments, 5 segments, 6 segments, 7 segments, 8 segments, 9 segments, or 10 segments. According to some examples, the segments may be movable along with their corresponding heat absorbers. Each possibility represents a separate example of the disclosure.

According to further examples, the segments may be positioned side by side. According to certain examples, the segments may be arranged in the form of a plier or tweezers. According to alternative examples, the segments may be arranged in the form of a camera shutter.

According to further examples, the at least one of the at least two segments may be configured to move, providing an opening through which the protrusion of the piercing element may be facilitated. According to some examples, the extension and the retraction of the piercing element may be configured to induce movement of at least one of the at least two segments of the thermo-conductive member. According to some examples, the piercing element extension may be configured to provide the opening in the thermo-conductive member. According to other examples, the piercing element retraction may be configured to facilitate the thermo-conductive member opening occlusion (closure).

According to further examples, the thermo-conductive member may be in contact with at least one temperature sensing means.

According to other examples, the skin thermo-conductive member may include an opening through which protrusion of the piercing element (the lancet/needle) may be facilitated. According to additional examples, the TEC element is configured to provide an opening through which protrusion of the skin piercing element is facilitated. According to additional examples, the TEC element/s is configured to provide an opening through which protrusion of the skin piercing element may be facilitated without removing the thermo-conductive member from the skin before, during and after skin piercing procedure.

According to other examples, the thermo-conductive member can be the TEC element itself with a permanent opening to be directly in contact with skin surface before and during skin puncturing. According to further examples, the thermo-conductive member can be two or more TEC elements with a little space between them for needle passage into the skin without removing the TEC elements from the skin. According to further examples, the thermo-conductive members which are in fact TEC element/s can be cooled and/or heated.

According to some examples, each thermo-conductive member may include a part which is movable heat absorber, coupled to the segment of the thermo-conductive member and configured to move along with the segment.

Without being limited by any specific theory or mechanism of action, according to some examples, the advantage of using the thermo-conductive member/s including the movable segments and not the thermo-conductive member/s, including a permanent opening is that during cooling and/or heating of the skin target area, the segments may be in their closed position, directly contacting the whole surface of the skin target area which allows cooling and/or heating the skin piercing point itself, where pain receptors that may directly sense the lancet/needle pricking should be effectively blocked by cryo-local-anesthesia. Therefore, using a thermo-conductive member/s including the segments provides an efficient and direct cryo-local-anesthesia of the skin target area.

The advantage of using the thermo-conductive member/s including the movable of at least two segments positioned side by side, compared to the camera shutter arrangement, is a reduced friction with the skin required to induce motion/movement of the at least one of the at least two segments providing the opening of the thermo-conductive members allowing the needle to pass between the segments and pierce the skin, thereby facilitating using a low power motor.

According to some examples, the piercing mechanism may include a spring loaded mechanism, a motorized mechanism, a linear actuator, a solenoid mechanism or any combination thereof.

According to further examples, the motorized mechanism may be configured to rotate in opposite directions. According to further examples, the motorized mechanism may include a screw with a nut rod configured to move on it forward and backward, pushing and pulling the needle into the skin and retracting the needle to its first position into the housing.

According to still further examples, the piercing mechanism may include a programmed circuit with a programmable microcontroller.

According to some examples, the piercing mechanism may include a depths setting mechanism, configured to determine the depth of the piercing element penetration into the skin target area, speed of penetration or both.

According to some examples, the operating control unit may be configured to trigger the extension of the piercing element by triggering means including mechanically operated means, electronically operated means or both.

According to further examples, the motorized mechanism may include a screw and a nut. According to still further examples, the motorized mechanism may be configured to push the piercing element by rotating the nut on the screw clockwise, thereby, pressing with the hub of the needle on one of the movable/retractable segments in contact with the skin, which leads to opening a tiny passage between both segments while a spring is expended, so that it allows the needle moving forward piercing the targeted skin site.

According to some examples, retracting the needle is triggered by the operating control unit configured to transfer a signal to the motor, reversing the motor rotation direction. According to yet further examples, upon retraction of the piercing element into the housing, the spring is constricted, which closes the passage between the segments to be as one whole segment. According to some examples, the control unit may be configured to monitor the piercing element penetration depth.

According to some examples, the piercing element may include a blood lancet or a needle. According to some embodiments, the lancet or the needle may be of different dimensions (gauges). According to some examples, the needle may be a hypodermic needle made of a tiny pipe with a sharpened end. According to some examples, the piercing element may be disposable. According to other examples, only the lancet or the needle of the piercing element may be disposable. According to some examples, the piercing element may include a cartridge containing a plurality of disposable lancets or needles.

According to some examples, the lancet or needle may be custom-designed dedicated only for the present blood sampling device and may include mechanic, optic, electronic or any other detecting means.

According to further examples, the piercing element may include materials selected from a group consisting of plastic, metal, Teflon, and a combination thereof.

According to some examples, the piercing element may include a protective member, configured to cover the lancet or the needle. According to further examples, the protective member may be removed by the user before the lancing procedure and after inserting the lancet into the housing of the blood sampling device. According to further examples, the piercing system may be configured to facilitate an insertion of the piercing element without removing the protective member. According to yet further examples, the protective member may be removed from the piercing element after the insertion of the piercing element into the housing of the blood sampling device. The protective member may include a gripper which allows gripping the lancet, thereby removing the lancet/needle by moving the gripper forward until it hits a mechanical blockage which allows picking it up and taking it out of the housing, which allows maintaining the lancet/needle sterilized.

According to further examples, the base of the lancet (the hub) may be in contact with a sensing means coupled to the blood sampling device, which may indicate to the user when the lancet is inside the housing. The sensing means may be connected to the programmed microcontroller operating control unit, which may be configured to provide indication/s including instructions to end users of the blood sampling device. For example, if the existence of a lancet/needle is not indicated, the microcontroller may be configured to prevent skin piercing and blood sampling.

According to some examples, the blood sampling device may include at least one temperature sensing means, configured to detect and monitor temperature at or in the vicinity of the thermo-conductive member. According to further examples, the temperature sensing means may be configured to communicate with the operating control unit microprocessor. According to further examples, at least one thermo-conductive member may directly or indirectly contact the at least one temperature sensing means. According to additional examples, the blood sampling device may further include at least one temperature sensing means connected to the thermo-conductive member/s, wherein the temperature sensing means may be configured to monitor the temperature of the thermo-conductive member. According to other examples, each heat absorber of the blood sampling device may include a temperature sensing means.

According to some examples, the blood sampling device may include a timer, configured to measure a cooling time, a heating time or both. According to further examples, the timer may be configured to communicate with the operating control unit. According to additional examples, the blood sampling device may include a proximity detector disposed at the thermo-conductive member, configured to determine a distance of the thermo-conductive member from the target skin area. According to further examples, the blood sampling device may indicate if the device contacts the skin. The indication may be achieved by the temperature sensing means configured to measure the temperature of the thermo-conductive-member/s-segments configured to be raised when touching the skin. If the temperature detected by the sensing means is not raised the skin is not in contact with the thermo-conductive member/s-segments. If the temperature sensing means detects that the temperature of the thermo-conductive suddenly rises several degrees C, it means that it detects that the skin is in contact with the thermo-conductive member.

According to some examples, the blood sampling device may be powered by at least one electrical source. According to additional examples, the electrical source may be a DC battery, a rechargeable battery, external electric power supply, or a combination thereof. According to further examples, the device may include a charger, a transformer or an electrical cable plug-in to the external power supply.

The present disclosure also provides a blood monitoring system. The blood sampling system may include a blood sampling device including a thermo-conductive member configured to cool and heat a skin area before, during and after skin pricking. The blood sampling device includes a piercing system, including a piercing element configured to pierce the target skin area and a mechanism for extending and retracting the skin piercing element. The blood sampling system includes a blood test meter.

According to some examples, the blood test meter may be configured to monitor blood glucose. According to other examples, the blood test meter may be configured to measure other blood values, such as, but not limited to, hemoglobin, ketones, blood coagulants, cholesterol or a combination thereof.

According to some embodiment, the system includes at least one thermo-electric cooling (TEC) element. According to other embodiments, the skin thermo-conductive member/s-segments include at least two TEC elements.

According to some examples, the blood sampling system further includes a heat absorber or an insulating partition between the blood sampling device and the blood test meter in order to avoid heating of the blood test meter. According to further examples, the thermo-electric cooling element (TEC) may be configured to cool the heat absorber partition.

According to further examples, in order to allow efficient reuse of the blood sampling device, the heat absorber may be configured to be cooled to a room temperature or slightly above room temperature in a few minutes (for example, 1 to 2 minutes). This is facilitated by cooling the heat absorber partition by the thermo-electric cooling element/s.

According to further examples, the temperature sensing means may be configured to communicate with the operating control unit.

According to yet further examples, the blood test meter may be housed in the same housing with a separated blood sampling device, including separated by a thermally isolated surface/partition to avoid heating the blood test meter by the heat absorber, or the high temperature radiant from the heat absorber to avoid the risk of providing inaccurate blood value results.

According to further examples, the housing of the integrated blood test meter and the blood sampling device may contain ventilation opening/s to cool the heat absorber.

According to still further examples, the system further includes a temperature sensor, configured to monitor temperature at or in the vicinity of the blood test meter. According to some examples, the temperature sensing means may be configured to communicate with the operating control unit.

The blood sampling system may be configured to initiate supplementary cooling of the blood test meter upon reaching the predetermined temperature, to transmit an overheating indication to the user, so that the user may wait until the temperature is reduced, until the device is ready to be used or both. Each possibility represents a separate example of the invention.

If the blood test meter temperature increases above a predetermined temperature, such as, but not limited to, 39°C, 38°C, 37°C, 36°C or 35°C, the heat absorber may be cooled down by the TEC element configured to cool or heat the thermo-conductive member, by additional TEC element connected to an additional heat absorber, by an external fan or by the fan of the docking station, as described herein-below. Each possibility represents a separate example of the invention.

According to some examples, the heat absorber includes temperature sensing means. According to some examples, the temperature sensing means may be configured to communicate with the operating control unit.

According to some examples, the blood sampling system further includes at least one disposable blood test strip. According to other examples, the system includes a plurality of blood test strips. According to other examples, the test strips used by the blood test meter are specifically designed and may include detecting means, such as optical detection means, and/or mechanic or electronic detecting means, and the like.

According to further examples, the blood test strips may be enclosed in a cartridge. The blood test strip cartridge may be a single test strip cartridge or a multi-strip test strip cartridge. According to still further examples, the system may include a mechanism for manually or automatically loading blood test strips. According to yet further examples, the blood test strips may be loaded into the testing region in the blood test meter, one at a time.

According to still further examples, the blood test meter may include a mechanism for automatically ejecting the blood test strip from the system after the test is complete. The blood test strip cartridge may be preferably encased in a transparent material or otherwise includes a visual indicator so that the number of the remaining strips which are available for testing can be determined.

According to a certain example, the entire blood test strip unit may be disposable, and is discarded when all the strips have been used.

According to another example, the blood test strip cartridge may be configured to be removed and replaced with a new test strip cartridge which is loaded with blood test strips.

According to further examples, the test strips may be flexible. According to additional examples, the test strips may be made of paper, or other flexible material, including chemical means, or having features used in blood test strips, rolled on at least one drum like in a film in a camera.

According to some examples, the blood test strips quantitatively measure glucose in blood samples. According to other examples, the blood test strips quantitatively measure other blood values, such as, but not limited to, hemoglobin, ketones, blood coagulants or a combination thereof.

According to additional examples, the blood test strips may be configured to be used only with the present disclosure blood monitoring system.

According to other examples, the blood sampling device may be configured to be used only if the integrated blood test meter is also used.

According to some examples, the blood test strip may be configured to transfer the blood sample to a reaction chamber of the blood test meter through capillary action. According to additional examples, the blood test meter comprises electronic components and/or any chemical material/means configured to perform blood values monitoring, including but not limited, blood glucose monitoring.

According to some examples, the blood test strips may measure Cholesterol or other blood parameters/values which requires large volume of blood samples. In such blood test/s, the present disclosure blood sampling device may be used for fingertip piercing that may include a heating step, at temperatures not higher than 10-35 degrees C, before and/or during and/or after skin pricking.

According to some examples, the blood sampling system may include wireless communication means configured to transmit blood test meter readings to a remote user.

According to further examples, the wireless communication means may include Bluetooth, Wi-Fi, or a chip such as, but not limited to, Home Health Hub., or a sensing means including chemical means configured to detecting glucose that may be connected to a cell phone to be used as a BGM. Each possibility represents a separate example of the disclosure.

According to some examples, the wireless communication means may include a cellular phone, wherein the blood test meter readings may be directly transmitted to a cell phone and transmitted to a monitoring station or a server which may store the results. The communicated blood test meter readings may be evaluated by medical staff that may decide on diet, drug dosing, or exercise that may be communicated back to the user.

According to some examples, the blood test meter may be programmed with a disease management system, using any parameters required to define or calculate dosage of medications according to algorithms, so that readings/results, including insulin dosage can be directly transmitted to a cell phone, including an application configured to analyze and store the blood test meter readings.

According to additional examples, the blood sampling system may include communication means configured to allow communication between the blood sampling device and the blood test meter. For example, the blood sampling device may receive indication from the blood test meter regarding the availability of blood test strips.

According to further examples, the blood sampling system may further include audio or visual means, configured to provide an indication to the user, including end of cooling indication, start of piercing indication, end of piercing indication, end of heating indication or any combination thereof.

According to additional examples, the system may further include a separate display for the blood sampling device, configured to present the blood test results and the indications transmitted by the components of the system, such as end of cooling indication, start of piercing indication, end of piercing indication, end of heating indication, overheating of the blood test meter indication or any combination thereof. According to other examples, the blood sampling device may include in the housing Lexan Panel which includes indication means and buttons.

According to some examples, the system further includes calculating means according to specific algorithms for calculating medication dosage according to level of glucose, amount of carbohydrate and/or other required parameters, including providing programmed adjusted diet plan according to blood glucose levels.

According to additional examples, the system may include an integrated medication dispenser.

According to some examples, the blood sampling system may be powered by at least one electrical source. According to additional examples, the electrical source may be a DC battery, a rechargeable battery, external electric power supply, or a combination thereof. According to further examples, the DC battery may be configured to supply electricity to the blood sampling device, to the blood test meter or both. According to still further examples, the system may include a charger, a transformer or an electrical cable plug-in to the external electric power supply.

According to some examples, the blood sampling device, the blood monitoring system, or both may be coupled to a docking station system that may include: a cooling system configured to communicate with a microcontroller programmed operating control unit, the cooling system may be in contact with a segment/s or a disk made of thermal conductivity feature/s, a temperature sensing means connected with the cooling system or with a disk which communicates with the microcontroller operating control unit.

The microcontroller operating control unit may be configured to control the cooling and the charging of the coupled device. The docking station system may include electric power supply plug-in and/or at least one DC battery, a charger for battery charging. The docking station system may include a cooling system based on at least one TEC element that its cold end is connected to a thermal-conductive member, while its hot end is connected with a heat absorber.

The docking station system may include a transformer, a heat absorber connected to the hot plate of the TEC, a fan for cooling the heat absorber, indication light/s, communication means such as, but not limited to, Bluetooth, Wi-Fi, a chip or a cellular phone, configured to allow communication between the blood sampling device and the blood test meter, a display, configured to present the blood test meter readings, or any combination thereof. Each possibility represents a separate example of the disclosure.

According to a certain example, inserting the device, the system or both into the docketing station, allows charging the battery of the device, the system or both. According to another example, inserting the device, the system or both into the docket station provides cooling of the thermo-conductive member by the contacting TEC element of the docket station, such the device, the system or both are ready for use after disconnecting from the docket station.

According to some examples, the blood sampling device is configured to draw blood samples from non-fingertips alternate sites skin target area, wherein the blood samples are sufficient for standard blood glucose monitoring.

According to some examples, the alternate site may be palm, arm, thigh or hip.

According to further examples, the blood sampling device may be configured to increase the power and velocity of blood flow in capillaries located in the alternate site skin, wherein the device is contacting the alternate site target skin area.

According to some examples, the blood sampling device cooling and heating system increases the pressure and velocity of blood flow in the capillaries close to the puncturing site by producing high internal blood pressure by creating an effective blockage of blood flow due to the cooled constricted capillaries.

According to some examples, the blockage is removed by piercing the skin target area followed by a heating step, performed at predetermined temperature and time and piercing is performed. The heating step is configured to expend the constricted capillaries diameter, the accumulated high blood pressure is abruptly released, resulting in a blood flow boost that pushes forward diluted blood out of the puncturing site with high blood pressure and increased power and velocity.

According to some examples, sufficient volume of blood samples are obtained with essentially no lag time of glucose concentration reaching the cells of the alternate puncturing site, which allows detecting hypoglycemia or hyperglycemia in real-time, to be treated immediately.

Thus, the method of the present disclosure allows producing sufficient volume of blood samples from alternate sites comparable to fingertip SBGM.

According to some examples, the device, the system and the method of the present disclosure allow to obtain blood sample from an alternate site, wherein the volume of the sample is in the range from about 0.1 to about 50 µl, such as from about 0.1 to about 1 µl, from about 1 to about 10 µl, or from about 10 µl to about 50 µl. Each possibility represents a separate example of the disclosure.

According to some examples, before removing the blockage of blood flow, a skin piercing procedure is performed by the piercing mechanism, and where the micro-controller is configured to provide a signal which changes the voltage polarity applied on the TEC elements. The high blood pressure is then released in capillaries located around the blockage, a blood flow boost pushes the diluted blood at a high pressure and velocity to the cells of the punctured site, while the device is still in contact with the skin.

### SUMMARY OF THE INVENTION

In accordance with the present invention, there is provided a blood sampling device as defined in claim 1 and a blood sampling system as defined in claim 12.

Further advantages are achieved by the embodiments indicated by the dependent claims.

In addition to the exemplary aspects and examples described above, further aspects, examples and embodiments will become apparent by reference to the figures and by study of the following detailed description.

### BRIEF DESCRIPTION OF THE FIGURES

Exemplary embodiments are illustrated in referenced figures. Dimensions of components and features shown in the figures are generally chosen for convenience and clarity of presentation and are not necessarily shown to scale. It is intended that the embodiments and figures disclosed herein are to be considered illustrative rather than restrictive. The figures are listed below.
Fig. 1 schematically illustrates an isometric view of the blood sampling device, in accordance with an embodiment of the invention;
Fig. 2 schematically illustrates an isometric view of the blood monitoring system, in accordance with an embodiment of the invention;
Fig. 3 schematically illustrates an isometric view of the movable thermo-conductive segments (TCG) of the blood sampling device, in accordance with an embodiment of the invention;
Fig. 4a schematically illustrates an isometric view of the piercing element, including a protective cover, in accordance with an embodiment of the invention ;
Fig. 4b schematically illustrates an isometric view of the piercing element, without a protective cover, in accordance with an embodiment of the invention.
Fig. 5a schematically illustrates a top view of the blood sampling device, in accordance with an embodiment of the invention;
Fig. 5b schematically illustrates a cross-sectional view of the blood sampling device, in accordance with an embodiment of the invention;
Fig. 6a schematically illustrates a cross-sectional side view of the blood sampling device, wherein the movable thermo-conductive segments TCG are in a closed position during cooling and/or heating the skin, in accordance with an embodiment of the invention;
Fig. 6b schematically illustrates a cross-sectional side view of the blood sampling device, wherein the movable thermo-conductive segments TCG are in an opened position when the needle inserts into the skin and is retracted, in accordance with an embodiment of the invention;
Fig. 7 schematically illustrates a flow chart of an exemplary mode of operation of the blood sampling system shown in Figure 2, for measuring blood glucose;
Fig. 8 schematically illustrates a flow chart of an additional exemplary mode of operation of blood sampling system shown in Figure 2, for measuring blood glucose; and
Fig. 9 schematically illustrates a block diagram of an exemplary mode operation of the blood sampling system shown in Figure 2, for measuring blood glucose, in accordance with an embodiment of the invention.

### DETAILED DESCRIPTION

Reference(s) to "embodiment(s)" throughout the description which are not under the scope of the appended claims merely represent possible exemplary executions and are therefore not part of the invention.

The invention is defined by the appended claims.

The term 'TEC' as used herein refers to a thermo electric cooler unit based on the Peltier effect.

The term 'BGM' as used herein refers to blood glucose test meters such as glucometers that require substential blood volume samples in order to provide accurate glucose values.

The terms 'alternative sites', 'alternate sites' or 'other skin region or sites' as used herein refer to a skin site of the body other than fingertips, such as, but not limited to, an arm, a thigh, a palm hand, or inner fleshy side of fingers.

The terms 'piercing site' or ' skin target area' as used herein refer to a surface area of a skin of a user at the piercing point of the lancet/needle and in the vicinity of the intended piercing point.

The present description discloses a blood sampling device, such as, but not limited to, a SBGM device, and a blood sampling system configured to sample blood parameters, such as, but not limited to glucose, from alternate sites. The sampling device and the monitoring system are configured to increase the volume of blood samples by increasing the blood flow in capillaries located in alternate sites skin. The increased blood flow is achieved by a coooling step, configured to increase the inner blood pressure by constricting capillaries resulted in creating a blockage of blood flow while the blood keeps on flowing against the blockage, and to provide by said cooling step cryo-local-anesthesia as a function of prederemied time needed to cool the skin until no pain is felt in practical application, followed by a heating step after the skin is pricked, configured to remove the blockage of blood flow by expending fast the diameter of capillaries while the viscous blood is diluted , which from the one hand avoids the pricking pain and from the other hand it increases the power and velocity of blood flow resulted in avoiding the lag-time reaching blood glucose concentration into the cells of the skin, producing large volume blood samples similar to fingertips.

Embodiments of the present invention provide a blood sampling device configured to generate sufficient blood samples volumes from sites other than fingertips. Furthermore, the blood sampling device is configured to provide accurate blood glucose results in real time from non-fingertips alternate sites equivalent to fingertips by increasing the inner pressure and velocity of blood flow in capilaries at the alternate site, according to methods described herein, in accordance with some embodiments. According to some embodiments, the alternate sites may be hand palms, for example, where clinical tests have shown that the accuracy of glucose results is similar to fingertips.

According to some embodiments, the blood sampling device implements, imitates and simulates a method for increasing the pressure and velocity of fluid in a pipe line fluifly connected to an open tap. According to some embodiments, the smallest blood vessels (capillaries) located in alternate site skin are cooled, constrict and hence blocked while the blood keep in flowing against the blockage, producing high inner blood pressure in blood capillaries. After the skin is punctured, a heating step removes the blockage due to expending fast of the constricted capillaries to their normal diameter or more, the accumulated high blood pressure is abruptly released, providing a blood flow boost, thereby the diluted blood is pushed forward out of the puncture site at a high power, pressure and velocity, producing sufficient blood samples volumes at least comparable to fingertips piercing.

Additionally, the increased velocity of blood flow in capillaries located close to the puncture site avoids the typical lag time of 20-35 minutes of reaching glucose concentration to the alternate site skin cells, which allows getting accurate blood glucose level measurements from alternate site SBGM equivalent to fingertips. Thus, according to some embodiments, the blood monitoring method allows detecting hypoglycemia and hyperglycemia in real time using alternate site SBGM which allows immediate treating.

Therefore, embodiments of the present invention may replace fingertip SBGM meeting the requirements of medical staffs and the FDA for fingertip-free SBGM as a new standard of care.

According to some embodiments, the blood sampling device of the present invention includes a retractable thermo-conductive pad (RTCP) that includes at least two retractable segments or a thermo-conductive member/pad that includes at least two sliding segments. The terms retractable and sliding may be used interchangeably. At least one segment may be in connection with a temperature sensing means configured to communicate with a programmed microcontroller, referred to hereinafter as operating control unit (OCU) microcontroller. The RTCP is configured to contact a small part of a skin target area of an alternate site's skin, in order to cool directly the part of the skin to be pierced where pain receptors are located and should be blocked during skin piercing. The RTCP may be configured to reduce the skin temperature in a predetermined time needed until essentially no pain (or reduced pain) is felt in practical application, which avoids the need to detect when the temperature in the dermal skin layers drops to +8 C degrees or below, locally anesthizing the aletrnate site skin, preventing pain during skin piercing.

According to some embodiments, the piercing system may include a piercing element configured to pierce the skin target area and a piercing mechanism, mechanic or electronic, for extending and retracting fully automatically the skin piercing element. The piercing element and piercing mechanism may be configurred to communicate and be controlled and operated by the OCU microcontroller.

According to some embodiments, the present invention provides an integrated system, configured to pierce the skin, to obtain sufficient blood sample volumes, to get accurate blood glucose results using a blood test meter, wherein the device may include:
a. a skin piercing system to pierce the skin in order to open an opening in the skin to obtain blood samples for testing blood values;
b. one or more displays that may, communicate with the piercing system for displaying of blood test results;
c. cooling and heating system, and a method, configured to increase the inner pressure and the velocity of blood flow in capillaries, in order to produce a sufficient volume of blood samples from sites other than fingertips and to avoid or reduce in these sites the lag time of reaching glucose to the dermal skin layer;
d. at least one lancet/needle configured to pierce the skin and having an adjustable penetration depths (D) into the dermal skin layer where capillaries are located;
e. at least one blood test strip communicating with a blood test meter;
f. depth setting system configured to allow users to adjust the needle penetration depths into the alternate site skin;
g. button for operating the cooling-heating system;
h. indication means configured to indicate for example, battery status, device is ready for use, lancet is inside the device ready for use or no lancet inside, ready to conduct SBGM, skin is punctured, a plurality of malfunctions.
i. Optionally, the device may include telemedicine means, communicating with a smart phone for example, thereby providing an integrated telemedicine blood samling and BGM system.

According to some embodiments, the self blood glucose monitoring (SBGM) method described herein may include the following steps:
a. **Activating** - Pressing an activation button activates the cooling system configured to cool the skin thermo-conductive members - retractable segments to a predetermined temperature;
b. **Providing an indication to the user that the devivce is ready for use** - When the temperature of the retractable segments is reduced to a predetermined temperature, indication is provided to the user to perform blood testing;
c. **Automatic shutdown** - If within a predetermined time the automatic skin detection means do not detect skin, the cooling system is configured to turn off and shut down the device;
d. **If skin is detected** - If in a predetermined time the skin detection means of the device detect skin, the cooling system is configured to cool the skin located under the retractable segments for a predetermined time;
e. **Creating a blood flow blockage** - In several seconds, capillaries located in the skin in contact with the cooling segments constrict and blood flow in these capillaries is effectively blocked while the blood keep on flowing agaist the blockage. In order to improve the blood flow blockage, thereby increasing the capillaries blood pressure, the shape of the retractable segments configured to contact the skin may be concave with slightly pointing out edge such that the pointing out edge, configured to contact the skin, improves the blockage of blood flow;
f. **Improving the blood flow blockage** - Keeping the blockage by cooling the skin for a predetermined time, increases significantly the internal blood pressure in the capillaries. In order to increase further the internal blood pressure within capillaries located close to the puncture site, the blood flow blockage is improved by slightly pressing the cooling thermo-conductive-member/s-segments against the skin for a predetermined time;
g. **Blocking pain receptors by cryo-local-anesthesia** - Cooling and reducing the temperature of the dermal tissue during a predetermined time until no pain is felt in practical application provides an effective cryo-local-anesthesia, effectively blocking the pain receptors conductance, without measuring the dermal tissue temperature using implanted temperature sensor.
   It should be noted that direct measurement of the temperature in the dermal tissue under the skin is impractical due to the need to include a temperature sensing means inside the piercing element that penetrates into the dermal tissue for a fraction of a second and having a capablity to transmit signals from below the skin to the SBGM device;
h. **Skin piercing** - the microcontroller is configured to send an indication to the lancet to perform skin piercing, the lancet is configured to move forward to a predetermined distance, the lanct pushes the retractable segments which are configured to open a tiny passage between them, the lancet is configured to penetrate between the segments into the cryo-local-anesthtized skin, the lancet is configured to retract back into the device housing, and a spring is configured to close the tiny passage opened between the retractable segments;
i. **Heating and releasing the inner blood pressure** - Local heating the skin is performed for a predetermined time after piercing, expanding the constricted capillaries to at least their normal diameter, or even to a larger diameter, increasing blood flow in the capillaries close to the puncture site. Furthermore, heating the skin dilutes the viscous blood that becomes more watery. The blockage is tabruptly removed, the high inner blood pressure is released, boosting the blood flow in the cappilaries, providing two additive boosts to the regular blood flow: first boost due to removing the blockage of blood flow by releasing abruptly the accumulated high blood pressure in the capillaries by expanding the cappilaries, and a second boost due to diluting the viscous blood to a more watery blood. The blood flow boost results in increased power and velocity of blood flow directed to the skin puncture site and a large volume of blood sample is produced. This is not possible to to obtian without the cooling and then heating steps;
j. **Obtaining the blood sample** - the blood sampling device is removed from the skin target area and the blood is sampled by a blood test meter using a test strip. Due to the blood flow boost, the SBGM method described herein allows detecting hypo and hyper glycemia in real time and getting accurate blood glucose results equivalent to fingertip SBGM;
k. **Removing the blood samling device from the skin** - After indication is provided to the end user that the pricking process is completed, the end user removes the device from the skin, and due to heatig step the blood is pushed out immediately until a sufficient blood drop is produced.
l. **Cooling the heat absorber** - In order to allow reusing the blood sampling device within a short time of about 1 to 2 minutes, the blood sampling device's heat absorber needs to be cooled to room temperature as fast as possible. Cooling the heat absorber in about 2 minutes is performed by an automatic close loop process using the cold plate of the TEC. The heat absorber is cooled for predetermined time to a predetermined temperature. If the heat absorber temperature is reduced to room temperature, the cooling process is completed. If the temperature is still higher than room temperature, the cooling process continues until the heat absorber temperature is reduced.

According to further embodiments, the heating step described hereinabove is configured to increase the blood flow from deeper capillareis to the punctured skin site, accelerating healing of the skin injury by bringing more oxygen to the skin piercing site. Note the following citation from the American Pain Society Bulletine - Using Heat for Pain Treatment, Belanger, Alain-Yuan, "When local heat is applied to the skin, it causes more blood to flow into the areas. Heat affects the skin as well as the underlying tissues. When blood flow is increases to an area, it brings along oxygen and nutrients that helps to speed healing. The sensation of heat on the skin also provides something called an analgesic effect: it alerts the perception of pain so you don't hurt as much. Heat is best for injuries that are not in the acute phase."

According to some embodiments, another advantage of the SBGM device is that it prevents the pain of fingertip pricking, since fingertip pricking is replaced by alternate sites skin pricking which is painless due to the use of cryo-local-anesthesia.

The present invention uses a cooling system and process to cool the skin at the point to be pierced and around for a predetermined time required to achieve pain-free skin pricking. The predetermined time of pain-free skin piercing may be determined experimentally by testing alternate sites skin cooling using embodiments of the present invention blood sampling device.

According to further embodiments and without wishing to being limited by any specific theory, at least 5 or more seconds of cooling an alternate site skin target area using the SBGM device described herein provides pain-free skin piercing.

According to some embodiments, the device of the present invention may be a stand-alone unit. According to other embodiments, the device may be coupled to a blood test meter joined in a single housing as a dual purpose device. According to certain embodiments, the device may be coupled to a standard blood test meter using disposable test strips for testing blood values. According to further embodiments, the device may be removably coupleable to the blood test meter. According to some embodiments, the blood test meter may be configured to measure blood glucose levels. According to other embodiments, the blood test meter may be configured to test other blood values, such as, but not limited to, hemoglobin, ketones, cholesterol, blood coagulants or a combination thereof.

According to some embodiments, the device may be configured to provide a local cooling step of the skin puncture target area prior and during skin pricking procedure. According to other embodiments, the device may be configured to provide a local heating step of the skin target area, prior and/or after skin pricking procedure. According to yet further embodiments, the device may be configured to provide cooling and subsequent heating of the skin target area.

According to some embodiments, the device further includes a programmed OCU that includes a microcontroller configured to control and activate the device or system, including activating a timer configured to determine the time sufficient to decrease the temperature of the retractable thermo-conductive pad (RTCP) and then to increase the temperature of the RTCP. The OCU microcontroller may be further configured to trigger the extension of the piercing element, and to stop cooling or heating by stopping the operation of the TEC elements. According to additional embodiments, the device OCU microcontroller may be configured to trigger the extension of the piercing element upon receiving an indication/s with regard to the RTCP reaching a predetermined cooling time, at predetermined temperature, or both.

According to further embodiments, the RTCP may be configured to switch between decreasing the temperature of the RTCP, and increasing the temperature of the RTCP upon the retraction of the piercing element, or upon providing an indication with regard to the RTCP reaching a predetermined cooling time, a predetermined temperature, or any combination thereof. According to some embodiments, the RTCP may be configured to directly contact the skin target area, including the point of the skin to be pierced where pain receptors are located and should be blocked during skin piercing in order to prevent pain.

According to further embodiments, the RTCP may be configured to directly contact the point of the skin target area, including the point of the skin to be pierced where pain receptors to be pricked are located during cooling or heating of the skin target area. Cooling the skin under the RTCP is done according to predetermined temperature and/or predetermined time in order to facilitate capillaries constriction before and during insertion of the lancet, used as an effective blockage of blood flow which prevents blood flow through the constricted capillaries. Cooling for a predetermined time is configured to increase internal blood pressure within capillaries located below the blockage, which allows creating high internal blood pressure within capillaries in the dermal tissue of the skin in the vicinity of the RTCP.

According to further embodiments and without wishing to being limited by any specific theory/method or mechanism of action, in order to improve the blockage of blood flow in capillaries, additional pressure may be applied by the device on the piercing site. According to still further embodiments, the shape of the RTCP may be concave where the edge of the pitch is slightly pointing out, configured to contact the skin and improve the blockage by increasing the internal blood pressure within capillaries and decreasing the time required for cooling and/or heating the skin.

According to further embodiments, in order to remove the blockage created by the constricted capillaries and to allow releasing the accumulated high blood pressure abruptly, providing a boost to the velocity of the blood flow after piercing, a local heating step may be provided for a predetermined time and/or to a predetermined temperature. The heating provided expands the constricted capillaries to at least their normal diameter, or even to a larger diameter, and dilute the viscous blood passing through the capillaries, boosting the blood flow to the incision.

According to R. Jelnes, Dan Med Bull 1988 August; 35(4) "at 43-44-45 degrees C the cutaneous blood flow rates increased from 12 to 50 ml (100 g)·min⁻¹", i.e., blood flow was increased by about 400%, which in the present invention may equalize the velocity of blood flow of alternate site to fingertips.

According to further embodiments, the blood samplig device may include:
a. an operating button;
b. a button to adjust the penetration depth of the lancet;
c. a button to adjust the time indication when blood glucose should be tested;
d. indication means to notify the user when to test blood glucose;
e. indication means indicating the battery status;
f. indication means indicating that the device is ready to use;
g. indication means indicating that the lancing procedure is completed;
h. indication means indicating to the user when to test blood glucose; and indication means indicating whether the lancet/needle is inside the device;
i. (Optional) music playing means configured to play music when the device contacts the skin and/or during skin piercing. The provided music is configured to calm people who are afraid of skin piercing and is configured to increase compliance of embodiments of the present invention SBGM device.
j. According to further embodiments, the piercing device is configured to detect the skin by a skin detection means. If after a predetermined time the device does not detect a skin, the device is configured to turn-off. If the device detects the skin within the predetermined time, it continues the process until the skin piercing is completed and an indication is then provided to the user to remove the device from the skin and sample the blood using a test strip and a BGM.

According to further embodiments, the blood sampling device may communicate with any blood test meter, or BGM.

According to further embodiments, the programmed OCU of the blood sampling device may communicate with the OCU of the blood test meter or BGM.

According to further embodiments, the RTCP may be configured to locally cool the piercing site for a predetermined time for increasing the blood pressure in the blocked capillaries and for blocking pain receptors by an effective cryo-local-anesthesia described hereinabove.

According to some embodiments, the TEC includes a cold end, configured to cool the RTCP and a hot end, configured to heat the RTCP. According to additional embodiments, the cold end and the hot end are interchangeable.

According to some embodiments, the shape of the part of the RTCP which is in contact with the skin may be straight, concave or convex, and/or a thin plate mounted around the movable segments. Each possibility represents a separate embodiment of the invention.

According to some embodiments, after piercing is performed while the piercing site is blocked by cryo-local-anesthesia, and until the device is removed from the skin, a heating step of the skin under the RTCP may be performed, without removing the RTCP from the skin. The heating step may be performed by reversing the poles of the TEC unit/s, resulting in:
a. an increase of the temperature of the dermis skin layer configured to expand immediately the constricted capillaries to their normal, or to a higher diameter, providing a blood flow boost.
b. a dillution of viscous blood within capillaries in the vicinity of the puncturing site configured to increase the power and velocity of blood flow within the capillaries, producing sufficient blood sample volumes from typically thick skin and capillaries located deeper under the skin in alternate sites and weak blood flow of diabetes patients.
c. an improved healing time of the injury after skin piercing due to heating and supplying more oxygen to cells of the injured skin.

Without wishing to be bound by any specific mechanism of action, heating of the skin decreases the viscosity of blood, thereby increases the velocity of the more watery blood flow in the capillaries, which in combination with the high blood pressure accumulated during the cooling step results in the desired increased volumes of blood samples allowing accurate blood glucose results from alternate site SBGM equivalent to fingertip SBGM.

According to some embodiments, the device further includes at least one thermo-electric cooling (TEC) element in thermal contact with the RTCP, wherein the TEC element may be configured to cool and/or heat the RTCP.

According to some embodiments, the device includes at least two TEC elements. According to an exemplary embodiment, the device includes four TEC elements. According to some embodiments, the thermoelectric cooling element is powered by at least one electric power source. According to additional embodiments, the electric power source may be a DC battery that may be rechargeable, an external electric power supply, or a combination thereof.

According to additional embodiments, the heat absorber may be movable/retractable, i.e., each thermal retractable segment may be connected to at least one TEC that its hot plate may be connected to a separate heat absorber such that two retractable segments may have two retractable heat absorbers.

According to some embodiments, the heat absorber may be connected to a temperature sensing means configured to measure its temperature and to communicate with the microcontroller. The temperature sensing means may be configured to detect a predetermined temperature of the heat absorber and to provide the data to the microcontroller.

According to further embodiments, at least one temperature sensing means may be adapted to at least to one retractable segment which communicates with the microcontroller .

According to further embodiments, a temperature sensing means, may be located in the compartment of the blood test meter or BGM, configured to communicate with the programmable OCU, which may also communicate with the BGM or with other blood test meter/s.

According to some embodiments, a closed loop cooling system may include a timer configured to measure the time of local cooling of the skin for a predetermined time, that may be five seconds or more for example, before the skin is pierced.

According to some embodiments, the RTCP may be cooled to a temperature from about +10 to about -10 °C. According to further embodiments, the RTCP may be cooled to a temperature from about +10 ° C to about -5 °C. According to other embodiments, the retractable thermo-conductive pad (RTCP) may be cooled to a temperature from about +5 to about 0 °C. According to additional embodiments, the thermo-conductive member may be cooled to a temperature from about 0 to about -5 °C. According to further embodiments, the thermo-conductive member may be cooled to a temperature from about -5 to about -10 °C. According to some embodiments, the thermo-conductive member may be cooled to at least +10 °C. According to additional embodiments, the thermo-conductive member may be cooled down to not less than -10 °C.

According to further embodiments, the RTCP may be heated to a temperature from about +20 °C up to about +50 °C for a predetermined time. According to some embodiments, the RTCP may be cooled by vapor-compression refrigeration. According to further embodiments, the RTCP is readily-removable from the device and cooled or heated not with the device and later on adapted to the device.

According to some embodiments, the RTCP may be made of thermally-conductive materials. According to further embodiments, the thermo-conductive member may be removed from the device and cooled externally. According to some embodiments, the RTCP may include a heat absorber, configured to accumulate the excess of heat.

According to some embodiments, the RTCP does not include TEC unit/s. Accordingly, the skin piercing device that includes an RTCP may be connected to external cradle docking station configured to provide a cooling system used to cool the RTCP to a predetermined temperature.

The piercing system is configured to be ready for use when it is removed from the docking station.

The piercing system may include: at least one TEC in contact with a thermally-conductive pad; an electronic circuit; a programmed microcontroller; a plug-in connection to external power supply and/or at least one DC battery; a transformer; a charger for battery charging; a cooling system based on at least one TEC module; a heat absorber in contact with the hot plate of the TEC; a fan for cooling the heat absorbent; the fan can additionally cools the heat absorbent; indication light/s; a separate display placed within the housing of the cradle, communication means, such as a cell phone or a Bluetooth chips, configured to download and send wireless personalized reports to medical staffs;

The piercing system may include a connection with caregivers, including communication means that allow communication between the BGM the lancing system and the cradle and viewing glucose data on a PC or a cell phone;

The piercing system may include a timer configured to provide indications when to test glucose; when the BGM integrated with the lancing system is placed into the cradle, when its battery is charged and when the disk of the integrated lancing system is in contact with the cooled disk of cooling system of the cradle. Thereby, the cradle docking station may reduce 20-30 seconds of waiting until the retractable segments are cooled.

According to some embodiments, the RTCP may be disposable. According to other embodiments, the RTCP may be covered by an interchangeable thermal-conductive disposable cover. The interchangeable cover may be disposable, removable, thin and flexible cover and may be made of thermo-conductive materials such as, but not limited to, aluminum foil to avoid infection.

According to some embodiments, the interchangeable cover may be sterilized after blood sampling, thereby the blood sampling device may be configured to be used by more than one patient safely.

According to some embodiments, the RTCP may be configured to provide an opening through which a protrusion of the piercing element may be facilitated. According to further embodiments, the RTCP includes at least two segments. According to some embodiments, the RTCP may include a plurality of segments, for example, 2 segments, 3 segments, 4 segments, 5 segments, 6 segments, 7 segments, 8 segments, 9 segments, or 10 segments. Each possibility represents a separate embodiment of the invention. According to further embodiments, the segments may be positioned side by side. According to certain embodiments, sliding segments may be arranged in the form of pliers or tweezers, closed at their first position. According to alternative embodiments, the plurality of segments may be arranged in the form of a camera shutter.

According to further embodiments the at least one of the at least two RTCP segments may be configured to move, providing an opening through which the protrusion of the piercing element may be facilitated. According to some embodiments, the extension and the retraction of the piercing element may be configured to induce movement of at least one of the at least two segments of the RTCP.

According to some embodiments, the piercing element extension may be configured to induce an opening in the RTCP. According to other embodiments, the piercing element retraction is configured to facilitate the RTCP closing.

According to other embodiments, the RTCM includes an opening through which protrusion of the piercing element is facilitated. According to additional embodiments, the TEC element is configured to provide an opening through which protrusion of the skin piercing element is facilitated.

According to some embodiments, the RTCP or a part thereof may include a movable heat absorber, coupled to the segment of the RTCP and configured to move along with the segment.

Without being limited by any specific theory or mechanism of action, the advantage of using the RTCP including the segments and not the retractable thermal pad, including a permanent opening for needle passage is that during cooling and/or heating of the skin target area, the segments may be in their closed position, directly contacting the whole surface of the skin target site which allows cooling and/or heating the skin surface piercing site itself where the needle is configured to be inserted and where pain receptors are located and should be blocked by cryo-local-aersthesia at mimimum time needed until essentially no (or reduced) pain is felt at temperature of +8 degrees C or below, and not the area in the vicinity thereof. Therefore, using a RTCP that includes retractable segments allows a more efficient cooling and/or heating of the site of the skin to be pierced.

The advantage of using a RTCP including retractable segments positioned side by side, compared to a camera shutter arrangement, is a lower force (and therefore a reduced power motor) required to induce its motion.

According to some embodiments, the blood sampling device may include one or more temperature sensing means configured to communicate with the operating control unit. The temperature sensing means may be a thermistor that measures temperature of the heat absorber, in proximity to the blood test meter, and the skin thermo-conductive member/s, or the movable thermo-conductive segments, or the TEC element/s connected with the temperature sensing means.

The thermistor may be connected in series with a fixed value resistor to a voltage reference, where the voltage across the thermistor may be fed to analog input of a microcontroller. The thermistor characteristics together with a PID may be used to set the temperature of the Unit Under Test (UUT) to a specific temperature. The thermistor may be configured to communicate with the microcontroller, configured to control and operate the blood sampling device.

According to some embodiments, the thermo-conductive member may include a heat absorber configured to accumulate the excess of heat. According to some embodiments the heat absorber may be in contact with at least one temperature sensing means configured to communicate with the programmable OCU microcontroller.

According to some embodiments, the piercing mechanism may include a spring mechanism, a motorized mechanism, a linear actuator, a solenoid mechanism or any combination thereof.

According to further embodiments, the motorized mechanism may be based on a screw with nut rod configured to move forward on the screw rod for inserting the needle into the skin, and backwards retracting the needle.

According to still further embodiments, the piercing mechanism of the blood sampling device includes a circuit with a programmable micro-controller used as an operating control unit.

According to some embodiments, the piercing mechanism includes a depths setting mechanism connected with the operating control unit, configured to determine several optional depths of the piercing element penetration into the skin target area, speed of penetration or both.

According to some embodiments, the piercing mechanism is configured to displace the piercing element by from about 0.2 mm to about 3 mm, or more. According to certain embodiments, the piercing mechanism is configured to displace the piercing element by from about 2 to about 2.5 mm or more.

According to some embodiments, the operating control unit is configured to trigger the extension of the piercing element by triggering means including mechanically operated means, electronically operated means or both.

According to some embodiments, the operating control unit is configured to adjust the piercing element penetration depth.

According to further embodiments, a motorized lancing mechanism may be free of side vibrations, configured to prevent additional damage to the skin compared to mechanical spring loaded lancing mechanism that may produce side vibrations that may increase the damage of skin injuries resulted in increasing the pain, thereby configured to reduce the pricking pain and the healing time of the pierced skin.

According to some embodiments, the blood sampling device includes an opening in the housing for inserting the lancet or the needle into the piercing element and for taking it out, wherein the piercing element may be disposable.

The blood sampling device may include sensing detection means, configured to detect if the lancet or the needle are inserted into the piercing element or not, and to transmit a corresponding indication to the user.

According to some embodiments, the blood sampling device operation may be blocked until the piercing element including the lancet and needle are inserted and the needle cover is removed.

According to some embodiments, the blood sampling device may detect if the thermo-conductive member is in contact with the skin, or not, using a temperature sensing means connected to the thermo-conductive member, or the TEC element, which detects immediately temperature raising of about 2 degrees C or more followed by providing an indication signal to the operating control unit.

According to some embodiments, the lancet used for skin piercing may be specific to the blood sampling device of the present invention.

According to some embodiments the blood test strips may be specific to the integrated blood test meter of the present invention.

According to some embodiments, a thermo-conductive plate may be configured to separates between the skin piercing mechanism including its heat absorber from the blood test meter (BGM), in order to prevent over heating the BGM. In addition, the housing of the blood sampling device may include a temperature sensor and ventilation opening/s to prevent over heating the blood test meter.

According to additional embodiments, the blood sampling device may further include at least one temperature sensing means. According to other embodiments, the TEC element/s may include temperature sensing means which communicates with the operating control unit. According to further embodiments, the temperature sensing means may be configured to monitor temperature/s of the thermo-conductive member/s-segment/s, or the TEC element surface contacting the thermo-conductive member.

According to further embodiments, the temperature sensing means may be configured to monitor the temperature in the vicinity of electronic components. According to further embodiments, the temperature sensing means may be configured to measure the temperature/s within the TEC element/s and the compartment of the BGM, the heat absorber and the RTCP, and to communicate the mesured temperatures to the microcontroller.

According to some embodiments, the blood sampling device may further include a timer, configured to measure the cooling time and/or the heating time or both, as well as to measure the predetermined time to insert the lancet into the skin, and the like.

According to additional embodiments, the blood sampling device may further include a proximity detector disposed at the RTCP, configured to determine its distance to the skin target area.

The present invention also provides a blood sampling system including the blood sampling device. The blood sampling system may include: a display for the blood test meter, or two displays, for the BGM and for the blood sampling device, a piercing system, including a piercing element configured to pierce the skin target area, a mechanism for extending and retracting the skin piercing element and a blood test meter.

According to further embodiments, the blood sampling device may include a Lexan panel that includes indication means and buttons, for packaging of components or parts of the device such as computers, servers and communication systems, TEC, heat absorber, etc. The Lexan may include display panel, keyboard connected with internal electronic components, microcontroller operating cotrol unit and isolation.

According to some embodiments, the blood test meter may be configured to monitor blood glucose values. According to other embodiments, the blood test meter may be configured to monitor other blood values, such as, but not limited to, hemoglobin, ketones, blood coagulants, cholesterol, etc., or a combination thereof.

According to other embodiments, the blood sampling device may be configured to test choleseterol values from fingertips which requires larger blood volume than typical blood samples provided by a heating step before and/or after skin pricking in order to dillute the viscous blood to be more watery. According to a preferred embodiment, the system may further include at least one thermo-electric cooling (TEC) element.

According to further embodiments, after the user removes the device from the skin the TEC may be configured to shutdown automatically. According to further embodiments, after the user removes the device from the skin (after that blood test is completed), the TEC element may be configured to cool by a closed loop process until the temperature of the heat absorber is reduced to the a predetermined temperature (to allow reusing the device in a short time of about 2 minutes and preventing the need to waiti about 15 minute until the heat absorbent cools to a room temperature passively).

In order to reduce the time needed for reusing the device, a closed loop process controlled by the microcontroller may be configured to reduce the temperature of the heat absorber by reversing the electric poles of the TEC element/s. Thereby, the heated plate of the TEC is coolled, which cools further the heat absorber. Each step of cooling the heat absorber is done for predetermined time and typically not more than about 10-12 seconds to avoid accessive heating of the TEC element/s.

In each cooling step the poles of the TEC are reversed (comparing to heating configuration), the heated side of the TEC is coolled and cools further the heat absorber in contact with it for a predetermined time. The temperature of the heat absorber is checked by a temperature sensing means. If the temperature did not reach the predetermined temperature the TEC restarts and it cools the heat absorber further for a predetermined time, and then the temperature of the heat absorber is rechecked. If the temperature of the heat absorber is dropped below the predetermined temperature, the closed loop process is completed and the device is ready for a reuse.

According to further embodiments, when the user presses the operating button of the device, and the temperature of the thermal-conductive member/s-segments reaches the required predetermined temperature, indication is provided to the user to conduct the blood test. If within a predetermined time the device detects the skin of the user - by detecting an abrupt increase of the temperature of the thermal-conductive member/s-segment, the device continues the lancing procedure. If after a predetermined time the device does not detect the skin of the user, it turns off the cooling system.

Without wishing to be bound by any specific theory or mechanism of action, the advantage of using the retractable segments from the perspective of inducing cryo-local-anesthesia on alternate sites is that the segments are held at a closed position during cooling and heating the punctured site of the skin, direcly cooling and/or heating the skin site to be pierced, which reduces to minimum the cooling time of reducing the skin temperature to the critical level of cryo-local-anesthesia.

According to additional embodiments, the device may further include a proximity detector disposed at the thermo-conductive member, configured to determine its distance from the skin target area.

According to some embodiments, the blood sampling device may include a chip with recorded music configured to be played during skin piercing. The played music may be an indication of skin in contact and for ongoing skin piercing. Furthermore, playing music during skin piercing may distract the mind of patients that may be afraid of skin piercing for the time period of cooling, piercing and heating the skin target area.

According to further embodiments, the blood sampling device's operating control unit may communicate with the blood test meter's operating control unit. According to some embodiments, the blood test meter may further includes wireless communication means configured to transmit blood test meter readings to a remote user, or to a computer, including a telemedicine system and an insulin dosage calculator application that calculates the required insulin dosage according to known algorithms and the patient blood glucose results.

According to some embodiments, the blood sampling device may be configured to draw blood samples from alternate site skin area, wherein the blood samples are sufficient for a standard blood glucose monitoring and the accuracy of glucose is at least similar to fingertip SBGM.

According to other embodiments, the blood sampling system is configured to draw blood samples from alternate site skin area, wherein the accuracy of glucose results obtained in real time are similar to fingertip SBGM.

According to some embodiments, the alternate site comprises arm, palm hand, thigh or hip, etc. According to additional embodiments, the system is configured to provide a quantitative measurement of glucose from less than about 0.4 to about 33.3 mmol/L.

According to some embodiments, the blood sampling device is configured to increase the pressure and velocity of blood flow in capillaries located close to the alternate site skin to be punctured, wherein the device is contacting the skin target area of the alternate site, due to two additive boosts: the heating step removes the blockage of blood flow and the accumulated high pressure is then released resulted in a blood flow boost directed to the punctured site through capillaries that were constricted by the preceding cooling step. The heating step enlarges immediately the diameter of capillaries to their normal size or even to a larger diameter, thereby, more blood may flow through the capillaries, and furthermore, the heating step dilutes viscous blood providing an additional blood flow boost.

According to another example not being part of the present invention a disposable piercing element including a blood lancet or a hypodermic needle which may include a protective member, configured to cover a lancet or a needle of the piercing element, wherein the piercing element is configured to be inserted into a blood sampling device without removing the protective member is provided for.

The protective member may include a grip member configured to facilitate removing of the protective member from the lancet or the needle, wherein the dispoable piercing element is configured to be inserted into the blood sampling device.

According to another example not being part of, the present invention a method for blood sampling based on increasing the pressure and velocity of blood flow in capillaries located near an alternate site skin area to be pierced. The method for blood sampling may meet the medical stuff and the FDA requirements for replacing fingertip SBGM is provided for.

The method for blood sampling may include:
- Cooling a thermo-conductive member/s-segments, via a control unit of a blood sampling device, to a predetermined temperature for a predetermined time;
- Indicating to the user that the device is contacting the skin;
- Cooling the skin at predetermined time;
- Triggering, via the control unit, a mechanism to extend a skin piercing element;
- Triggering, via the control unit, the mechanism to retract the skin piercing element after the skin target area was pierced;
- Heating the thermo-conductive member, via the operating control unit to a predetermined temperature for a predetermined time;
- Indicating to the user that the device may be removed from the skin, to allow blood flowing out from the incision site; and
- Cooling automatically the heat absorber by a closed loop process until it reaches room temperature which is needed to reduce the waiting time for the next blood test and the next TEC element/s operation compared to natural cooling of the heat absorber, which may take at least 15 minutes by natural cooling until it is possible to reuse the device.

The triggering steps may precede the heating step. The heating step may precede the triggering steps. Alternatively, , the method includes:
- Cooling a thermo-conductive member/s-segment/s, via an operating control unit of a blood sampling device, to a predetermined temperature;
- Triggering, via the control unit, a mechanism to extend a skin piercing element; and
- Triggering, via the control unit, the mechanism to retract the skin piercing element after the skin target area was pierced.

The method may further include a step of automatically moving segments of the movable/retractable thermo-conductive member/pad (RTCP) effected by extending and/or retracting the piercing element.

Cooling the thermo-conductive member may include activating a thermoelectric cooling (TEC) element.

Heating the RTCP may include reversing the polarity of the voltage applied to the TEC element.

Heating the RTCP may include activating the TEC element/s. Cooling the heat absorber may include reversing the polarity of the voltage applied to the TEC element.

The cooling step further allows creating a blockage of blood flow due to creating a blockage of blood flow by constricting of capillaries during a cooling step, while blood flow against the blockage keep on flowing, which produces high internal blood pressure increased from second to second. Additionally, during the cooling process is used as a cryo-local-anesthesia of the skin, cooling temperature below about +8 degrees C, which allows preventig the pain generated by skin piercing.

The heating step further speeds healing of the skin piercing injuries. The same heating process for expending fast the diameter of capillaries in order to remove the blockage of blood flow, also causes to the dillution of viscous blood resulting in increasig the velocity of lood flow, allowing capillaries bringing much more oxygen to the injured site with blood flow, thereby, faster healing of the incision in the skin target area to be injured.

The heating step further allows reducing the time required for performing the next blood test by reducing the time of cooling the heat absorber to a room temperature, compared to passive cooling, which is needed for reusing the device.

The heating step allows reducing the time required to wait before sampling blood with a test meter by about 8 seconds by natural heating which is needed for expending the constricted capillaries to their normal volume allowing blood going out of the skin.

Further provided is as an example not being part of the invention a method for obtaining a blood sample from a non-fingertip alternate site SBGM, including:
- Cooling down and reducing the temperature of the dermis and the epidermis skin layer of a puncturing site;
- Piercing the skin;
- Heating the skin surface of the puncture site; and
- Obtaining blood sample.

A blood test strip of a blood test meter may be used to sample the blood and within few seconds the blood test results are provided by the blood test meter.

The method allows increasing the inner pressure and velocity of blood flow in capillaries located in the vicinity of the alternate pircing site. The method allows increasing the internal pressure within capillaries located near the area to be pierced in alternate sites by cooling and releasing the accumulated high pressure by heating.

The accumulated pressure produced near the area to be pierced in alternate sites is released by removing the blockage of the constricted capillaries which avoids a waiting time of about 8 secconds until the blood is going out of the skin by natural heating of the skin.

The method allows increasing the blood sample volume obtained from the capillaries located close to the puncture site of alternate sites.

The method reduces (or avoids) the lag time of reaching glucose concentration to alternate site skin, thereby providing an accurate SBGM results in real time from an alternate site similar to figertips piercing also when blood glucose is changed rapidly.

The method allows the meeting the medical and FDA requirements for SBGM and may replace fingertips SBGM and may be a new standard of care.

Alternate sites may include, but are not limited to, the palm, hip, and thigh as a new standard of care, which may release diabetic patients from the painful figertips pricking, allowing diabetics to overcome noncomplaince problems of fingertip SBGM.

Some advantages and benefits of the blood sampling device of embodiments of the disclosed invention are:
a. Replacing fingertips SBGM by alternate site SBGM achieves pain-free piercing. Fingertips SBGM prevents using a cooling step as an anesthetic step, due to a high density of thermal fibers sensitive to thermal contact, while alternate sites have lower density of thermal fibers, which allows alleviating pricking pain by a cooling step, which is avoided by fingertip SBGM.
b. Replacing fingertips SBGM by alternate site SBGM reduces the pain associated with fingertips injuries after skin pricking. Fingertip's skin is very sensitive to piercing due to a high density of pain receptors, while alternate site's skin includes lower density of pain receptors and is less sensitive to piercing.
c. Reducing the waiting time for obtaining blood sample. The heating step reduces by at least 8 seconds the waiting time until a sufficient volume blood drop is sampled.
d. Reducing the healing time of the injured skin by a heating step. Rehabilitation of the skin injury after puncturing is accelerated by the heating step that brings more oxygen to the injured site due to the increased blood flow.
e. Shortening the waiting time for a reuse. After a blood test is completed, in order to reuse the device the heat absorber requires cooling to about a room temperature. Passive cooling the heat absorber may take about 10-15 minutes. According to the present invention, a closed loop process is provided, configured to cool the heat absorber to a room temperature in about 2 minutes.
f. Avoiding the lag time of reaching glucose concentration to the cells of alternate site's skin by alternate site SBGM, to be equivalent to fingertips SBGM. The present invention provides a method for obtaining blood samples by alternate site SBGM that includes cooling the piercing site prior to piercing and during the piercing and subsequently heating the puncture site.

Cooling the skin results in rapid constriction of capillaries which allows a complete, or almost complete, blockage of blood flow close to the puncture site, thereby increasing the internal pressure within the capillaries.

Heating the skin results in capillaries expansion, thereby removing the blockage and producing a blood flow boost. Therefore, upon piercing and the succeeding heating of the skin, the constricted capillaries expand and blood is ejected from the piercing site, releasing the pressure developed during the cooling step. The expanded capillaries and the blood flow boost avoids the glucose lag time of alternate sites and provides accurate glucose results in real time equivalent to fingertips SBGM and produces large volume blood samples, which meets the medical and FDA requirements for fingertip-free alternate site SBGM as a new standard of care.

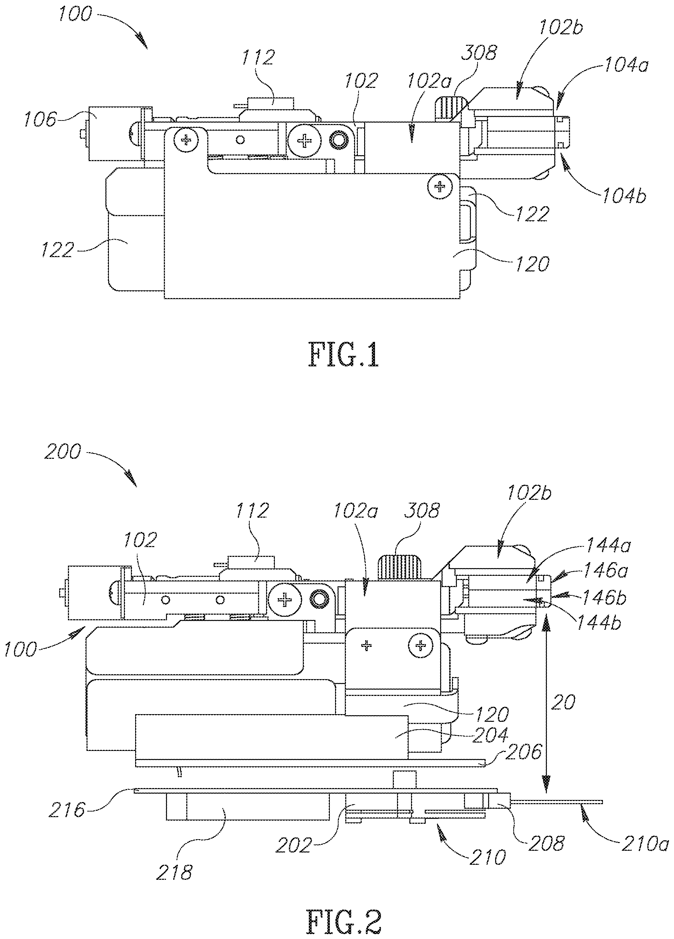

Reference is made to **Figure 1**, which schematically illustrates an isometric view of blood sampling device 100, in accordance to some embodiments. A blood sampling device 100 includes a piercing element housing/heat absorber 102, a control member 112, at least two thermo-conductive segments 104a and 104b, a motor 106, a battery housing 120 and a battery/ies 122.

Piercing element housing/heat absorber 102 contacts thermo-conductive segments 104a and 104b.

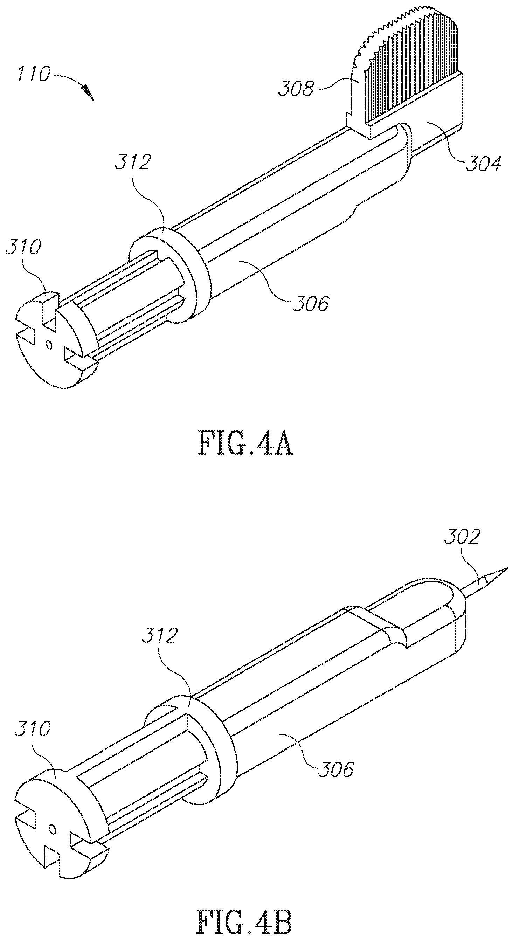

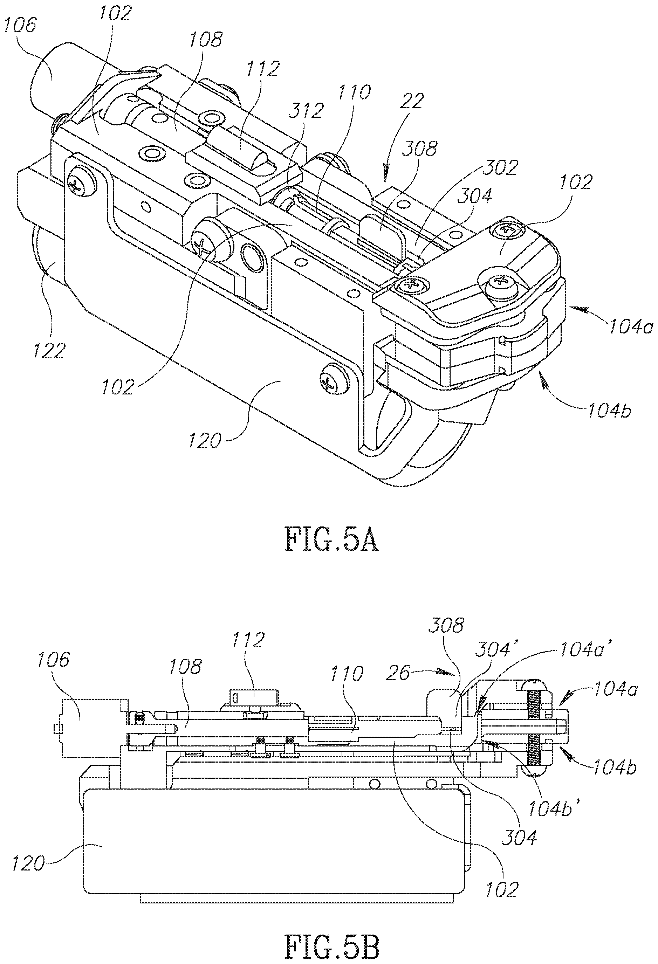

One or more of the components of piercing element housing/heat absorber 102 may be configured to accommodate a piercing element (not shown), illustrated in Fig. 5a and Fig. 5b and described further below. According to some embodiments, the piercing element (such as piercing element 110, shown, for example, in Figs. 4a-6b) may be accomodated in a piercing element housing which is not part of the heat absorber.

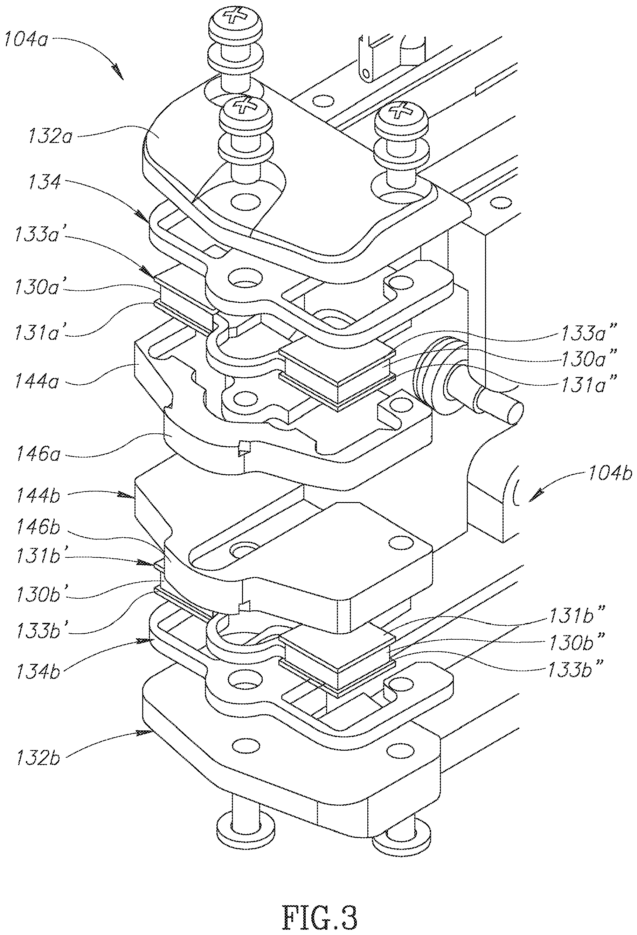

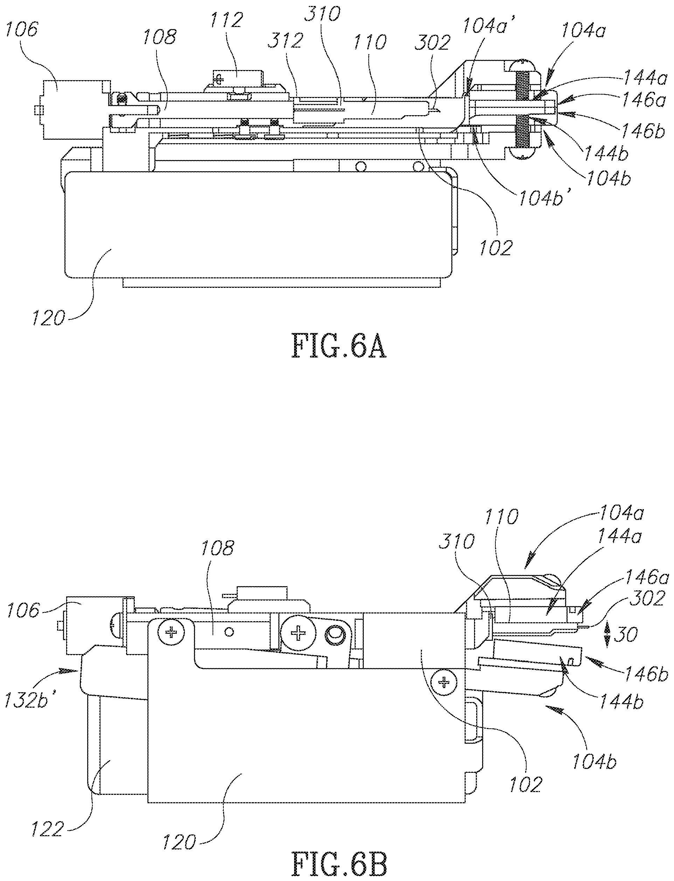

Thermo-conductive segment 104a includes thermo-conductive plate 146a and thermo-conductive segment 104b includes thermo-conductive plate 146b (shown Fig. 2). Plates 146a and 146b (shown Fig. 2) are configured to contact a user's skin and to facilitate cooling and heating of skin target area while the segments are in a closed position, as described in Fig. 3 herein further below. Thermo-conductive movable/retractable segments 104a and 104b are further configured to open a gap, allowing piercing element 110 (illustrated in Fig. 4) protrusion through the gap/passage, created between thermo-conductive segments 104a and 104b, illustrated in 30 (shown Fig. 6b).

Motor 106 is configured to contact piercing element housing/heat absorber 102 and is configured to facilitate the piercing element (not shown) extension configured to pierce the skin target area, and to retract, as illustrated in Fig. 6a and Fig. 6b.

Piercing element housing/heat absorber 102 is an elongated member configured to contact motor 106 with its proximal end and to contact thermo-conductive movable/retractable segments 104a and 104b with its distal end 102a and 102b.

Battery(ies) 122 is configured to provide power supply to motor 106, to a microcontroller (not shown) and to thermoelectric cooling (TEC) elements (not shown) disposed inside thermo-conductive segments 104a and 104b.

Reference is made to **Figure 2****,** which schematically illustrates an isometric view of blood sampling system 200, in accordance to some embodiments. Blood sampling system 200 includes a blood sampling device 100 coupled with blood glucose meter (BGM) 202. Blood sampling system 200 further includes a thermal/heat insulator 204 and a printed circuit board (PCB) 206 that includes blood sampling device 100 electronic control circuit. A thermal heat insulator 204 is disposed between a battery housing 120 of device 100 and PCB 206. BGM 202 is connected to PCB 206 at the other side thereof.

BGM 202 includes a test strip holder 208, a plurality of a disposable test strip 210, such as test strip 210a. BGM 202 is mounted on PCB 216 that includes further BGM 202 electronic control unit 218. Disposable test strip 210a extends from strip holder 208 and is configured to contact skin area after piercing procedure has been performed and a sufficient blood sample volume has been extracted. Upon contacting the extracted blood with test strip 210a, glucose concentration is evaluated by BGM 202, as extensively described in prior art, for example, in [K. Tonyushkina et al., J Diabetes Sci Technol. 2009 July; 3(4): 971-980]. Upon evaluating the glucose readings, test strip 210a may be removed from test strip holder 208 and disposed. Upon removing test strip 210a test strip 210b (not shown) may be extended from test strip holder 208 by hand, or by any mechanism known in art, such as, but not limited to, a conveyor, cam, driver with a cocking and release mechanism, or a cassette.

Since the system of the present invention is configured to allow cooling and heating of thermo-conductive members/plates 144a and 144b, temperature of the other elements of the system is configured be controlled by a temperature sensing means regulated by a microcontroller operating control unit (OCU). For example, temperature increase within BGM 202 above 39°C should be avoided in order to allow accurate glucose measurement. Additionally or alternatively, if the temperature of the thermo-conductive segments drops below - (minus) 10 °C, the microcontroller is configured to immediately stop the TEC operation.

Thermal heat insulator 204 is configured to absorb the heat accumulating during the cooling step of the glucose sampling procedure, in order to maintain the temperature in the vicinity of the blood test meter or the BGM below 39 °C. Thermal heat insulator 204 further allows contacting skin surface with thermo-conductive segments 104a and 104b without removing strip 210a from BGM 202 by providing effective space, denoted by arrow 20, between segments 104a and 104b and BGM 202 in blood sampling system 200.

Piercing element housing/heat absorber 102 is an elongated member that includes distal end sections 102a and 102b, which are in thermal contact with thermo- conductive members 144a and 144b.

According to some embodiments, the distal end of blood sampling device 100, that includes edges 146a and 146b of thermo-conductive plates 144a and 144b, is configured to extend from blood sampling system 200 further than BGM 202, in order to allow thermo-conductive segments 104a and 104b located outside the housing exert some pressure on the piercing site, preventing a contact of test strip 210a extending from BGM 202 with the user's skin surface, wherein such contact may impair a contact of thermo-conductive segments 104a and 104b with the piercing site.

Blood sampling device 100 (Fig. 1 and 2) includes a control member 112 and a piercing element grip 308 which are described in more details with reference to Fig. 5a below.

Reference is made to **Figure 3****,** which schematically illustrates an isometric exploded view of thermo-conductive segments 104a and 104b of a blood sampling device 100 (shown in Fig. 1) in accordance to some embodiments.

Thermo-conductive segments 104a and 104b include thermo-conductive plates 144a and 144b, respectively. Thermo-conductive plates 144a and 144b are outside the housing configured to contact user's skin in their closed position and to cool and/or heat the skin target area. Thermo-conductive plates 144a and 144b are configured to be thermally conducting and to allow decreasing and/or increasing temperature thereof. According to some embodiments, thermo-conductive plates 144a and 144b may comprise aluminum, copper, or other known heat conducting material. According to other embodiments, the thermo-conductive plates can be made of any thermally conductive material. According to additional embodiments, the thermo-conductive plates may be disposable. According to certain embodiments, the thermo-conductive plates may include interchangeable thermal conductive cover. Thermo-conductive edges 146a and 146b respectively are configured to contact the skin target area.

Cooling and/or heating of thermo-conductive plates 144a and 144b are enabled by a plurality of thermoelectric cooling elements (TEC elements) 130, using the known Peltier effect. The Peltier effect is sometimes referred to being a part of a thermoelectric effect, which is a direct conversion of temperature differences to electric voltage and vice versa. Thermoelectric cooling elements comprise two electrically connected plates made of different types of materials. A heat flux between the junction of the two plates is created when voltage is applied to plates 144a and 144b. Accordingly, TEC 130 is configured to transfer heat from one side of the element to the other, with consumption of electrical energy, depending on the voltage polarity.

Thermo-conductive segments 104a and 104b further include a plurality of insulators 134, configured to be in thermal contact with TEC elements 130 and configured to absorb the heat evolving during cooling of thermo-conductive plates 144a and 144b by TEC elements 130.

Thermo-conductive segments 104a and 104b further include panels 132a and 132b, respectively, configured to accommodate thermo-conductive plates 144a and 144b, insulators 134a and 134b and TEC elements 130 and to facilitate thermo-conductive segments movement, i.e. opening up and closing back to their initial closed state.

Thermoelectric cooling elements 130a' and 130a" are disposed in panel 132a between insulator 134a and thermo-conductive plate 144a. Thermoelectric cooling elements 130b" and 130b" are disposed in panel 132b, between insulator 134b and thermo-conductive plate 144b. Side surfaces 131a' and 131a" of thermoelectric cooling elements 130a' and 130a", respectively, are configured to contact thermo-conductive plate 144a, wherein side surfaces 133a' and 133a" of thermoelectric cooling elements 130a' and 130a", respectively, are configured to contact insulator 134a. Side surfaces 131b' and 131b" of thermoelectric cooling elements 130b' and 130b", respectively, are configured to contact thermo-conductive plate 144b, wherein side surfaces 133b' and 133b" of thermoelectric cooling elements 130b' and 130b", respectively, are configured to contact insulator 134b. Upon applying voltage to TEC elements 130a' and 130a" in a direction inducing heating of side surfaces 131a' and 131a" of the respective TEC elements, the heat is transmitted to contacting thermo-conductive plate 144a and the temperature of thermo-conductive plate 144a increases. Upon applying voltage on TEC elements 130b' and 130b" in a direction inducing heating of side surfaces 131b' and 131b" of the respective TEC elements, the heat is transmitted to contacting thermo-conductive plate 144b and the temperature of thermo-conductive plate 144b increases.

Contacting skin with heated thermo-conductive plates 144a and 144b allows heating the skin at the contact area and the capillaries disposed in the near proximity of the piercing site. Reversing the polarity of voltage applied on TEC elements 130a' and 130a", induces cooling of sides 131a' and 131a" of the respective TEC elements, and the respective cooling of the contacting thermo-conductive plate 144a.

Reversing the polarity of voltage applied on TEC elements 130b' and 130b" induces cooling of sides 131b' and 131b" of the respective TEC elements, and the respective cooling of the contacting thermo-conductive plate 144b. Contacting skin with cooled thermo-conductive plates 144a and 144b allows cooling the skin at the contact area and the capillaries disposed at or in the near proximity of the piercing site.

Changing the polarity of voltage applied on TEC elements 130a', 130b', 130a" and 130b" allows switching between cooling and heating of thermo-conductive plates 144a and 144b in less than 1 second. According to other embodiments, switching between cooling and heating of thermo-conductive plates 144a and 144b proceeds in less than 0.1 second .

Reversing the polarity of voltage applied on TEC elements 130a' and 130a", additionally induces heating of side surfaces 133a' and 133a" of the respective TEC elements, and reversing the polarity of voltage applied to TEC elements 130b' and 130b", similarly induces heating of side surfaces 133b' and 133b" of the respective TEC elements. The excess of heat accumulated at side surfaces 133a' and 133a" of TEC elements 130a' and 130a", respectively, is configured to be absorbed by insulator 134a. The excess of heat accumulated at the side surfaces 133b' and 133b" of TEC elements 130b' and 130b", respectively, is configured to be absorbed by insulator 134b.

According to some embodiments, the plurality of TEC elements 130 are configured to cool thermo-conductive plates 144a and 144b until the temperature of the thermo-conductive segments reaches the range from about -5°C to about 10°C. According to other embodiments, the plurality of TEC elements 130 are configured to cool thermo-conductive plates 144a and 144b for a predetermined time.

According to some embodiments, the plurality of TEC elements 130 are configured to heat thermo-conductive plates 144a and 144b until the temperature of the thermo-electric segments reaches the range from about 25°C to about 50°C. According to other embodiments, the plurality of TEC elements 130 are configured to heat thermo-conductive plates 144a and 144b for a predetermined time.

Reference is made to **Figure 4a****,** which schematically illustrates an isometric view of a piercing element 110, including a protective cover 304, in accordance to some embodiments.

Piercing element 110 that may be disposable includes a body 306 and circumferential elements 310 and 312, disposed around body 306. Body 306 is configured to accommodate a needle (as shown in Fig. 4b hereinbelow) and protective cover 304.

Circumferential element 310 is configured to contact actuator 108 (as shown in Fig. 5a hereinbelow), wherein the actuator is configured to exert pressure on circumferential element 310 and therefore to facilitate piercing element 110 exertion.

Circumferential element 312 is configured to exert pressure on at least one of thermo-conductive segments 104a and/or 104b (as shown in Fig. 6b, hereinbelow), to facilitate the opening up thereof, allowing piercing element 110 protrusion through the thermo-conductive segments upon extension of the piercing element.

Piercing element 110 further includes a removable protective cover 304. Protective cover 304 includes grip element 308, configured to allow convenient grabbing of protective cover 304. Piercing element 110 is configured to be inserted into piercing element housing/heat absorber 102 (shown in Fig. 1 and 2) without removing protective cover 304. Protective cover 304 is configured to be removed by pulling while holding cap cover at grip element 308 (as described in Fig. 5b hereinbelow).

Reference is made to **Figure 4b****,** which schematically illustrates an isometric view of a piercing element 110, without a protective cover 304, in accordance to some embodiments. Piercing element 110 includes a needle 302. Needle 302 is configured to pierce skin target area upon extending of piercing element 110, to obtain a blood sample.

Reference is made to **Figure 5a****,** which schematically illustrates a top isometric view of a blood sampling device 100, and provides a more detailed view of a piercing element housing/heat absorber 102 components and the components associated with it, in accordance to some embodiments.

Piercing element 110 is configured to be inserted into piercing element housing, which is shown herein as part of heat absorber 102. The direction of the insertion is denoted by arrow 22. Disposable piercing element 110 is further configured to be inserted into piercing element housing 102 without removing protective cover 304 including grip element 308, wherein the protective cover 304 is configured to secure needle 302. Protective cover 304 is configured to move by pushing forward grip element 308 until a mechanical stop. Thereby, protective cover 304 is removed from the piercing element. Pulling grip element 308 allows taking out protective cover 304 from piercing element housing 102.

Motor 106 is in contact with piercing element housing/heat absorber 102 and with actuator 108, disposed in piercing element housing 102. Actuator 108 is configured to actuate piercing element 110, which is also disposed in piercing element housing/heat absorber 102. Motor 106 is configured to actuate actuator 108 that further propels piercing element 110 towards thermo-conductive segments 104a and 104b. Upon contacting the segments, the pressure excreted by the piercing element, transmitted by actuator 108 driven by motor 106 is configured to induce opening of thermo-conducting segments 104a and 104b, thereby allowing piercing element 110 protrusion through the opening created between thermo-conductive segments 104a and 104b.

Motor 106 further includes a control member 112, configured to transform the motor radial motion to protrusion length of piercing element 110, thereby controlling the insertion depth of the needle (not shown) into the skin. According to some embodiments, blood sampling device 100 includes a microcontroller (not shown), configured to control motor 106 and control member 112 and configured to receive signals from control member 112 and to transmit signals to motor 106. Indications received from control member 112 may be processed by the microcontroller that may evaluate the protrusion length of piercing element 110 and control the predetermined insertion depth of the needle into the skin. The microcontroller may be configured to transmit indications to motor 106, when the predetermined insertion depth evaluated by control member 112 is achieved. Motor 106 is configured to reverse the direction of its radial motion upon receiving indications from the microcontroller, retracting piercing element 110 back into piercing element housing/heat absorber 102.

According to some embodiments, blood sampling device 100 further includes a temperature sensing means (not shown). The temperature sensing means may be coupled to thermo-conductive segments. According to some embodiments, the TEC elements (shown in Fig. 3 hereinabove) may be employed as temperature sensing means for skin contact detection. According to some embodiments, the temperature sensor is configured to detect temperature of thermo-conductive segments 104a and 104b. The temperature sensing means may be configured to detect skin temperature at the piercing site and to transmit the detected temperature to the microcontroller (not shown). According to some embodiments, upon cooling of thermo-conductive segments 104a and 104b to the predetermined temperature and/or for a predetermined time period, the programmed microcontroller operating control unit may be configured to transmit a signal to blood sampling device 100 providing an indication to the user that blood sampling device 100 is ready for blood sampling.

According to other embodiments, upon cooling of the thermo-conductive segments to the predetermined temperature and/or for the predetermined time period, the programmed microcontroller may be configured to transmit a signal indicating that blood sampling device 100 is ready for use. Upon contacting a skin target site, the temperature of thermo-conductive segments 104a and 104b momentarily increases, indicating a contact of blood sampling device 100 with the user's skin. The momentarily temperature increase may be detected by the temperature sensing means, transmitting a signal to the microprocessor, indicating that contact with the skin target area is made and that the next cooling step may be started.

According to some embodiments, blood sampling device 100 may further include a timer (not shown), configured to measure cooling and/or heating time and to transmit an indication to the microcontroller upon reaching the predetermined cooling and/or heating time. Upon reaching the predetermined cooling time, the microcontroller may be configured to trigger motor 106 radial motion, configured to rotate a screw in contact with motor 106 and a nut configured to move forward on the screw allowing extension of piercing element 110. The microcontroller may provide a signal to motor 106 to reverse its rotation direction, the screw may be configured to rotate to the opposite direction, and the nut may be configured to pull needle 302 out of the skin back into piercing element housing/heat absorber 102 to its original position. Upon reaching the predetermined heating time, the microcontroller may be configured to transmit a signal indicating that thermo-conducive segments 104a and 104b may be removed from the skin target site.

Reference is made to **Figure 5b****,** which schematically illustrates a cross-sectional side view of a blood sampling device 100. Blood sampling device 100 includes a piercing element 110, that may be disposable, inserted in a piercing element housing/heat absorber 102, while the piercing element is covered with a protective cover 304 and with a grip element 308. A space left between an edge 304' of cap 304 and a side 104a' of a retractable-thermo-conductive segment 104a and a side 104b' of a thermo-conductive segment 104b allows manual removing protective cover 304 from piercing element 110 by holding protective cover 304 at grip element 308 and pushing it towards the thermo-conductive segments until hitting a mechanical blockage in the direction denoted by arrow 26. At that stage, protective cover 304 is removed from piercing element 110, revealing needle 302 (not shown), allowing piercing device needle 302 to pierce the skin target area.

Reference is made to **Figure 6a****,** which schematically illustrates a cross-sectional side view of a blood sampling device 100, wherein thermo-conductive segments 104a and 104b are in a closed position, and to **Figure 6b****,** which schematically illustrates a cross-sectional side view of blood sampling device 100, wherein thermo-conductive segments 104a and 104b are in their opened position 30, in accordance to some embodiments.

In their closed position, a side of a thermo-conductive plate 144a contacts a side of a thermo-conductive plate 144b and distal end edges 146a and 146b of thermo-conductive plates 144a and 144b respectively contact the skin target area. A piercing element 110 is disposed inside a piercing element housing/heat absorber 102 in its retracted position. Piercing element 110 includes a needle 302, wherein needle 302 does not contact thermo-conductive segments 104a and 104b. A circumferential element 310 of piercing element 110 contacts an actuator 108. Upon activating motor 106, actuator 108, contacts piercing element 110, inducing piercing element 110 protrusion towards thermo-conductive segments 104a and 104b until contacting edges 104a' and 104b' of thermo-conductive segments 104a and 104b, respectively, with circumferential element 312. Circumferential element 312 of piercing device 110 in its extended position, exerts pressure on edges 104a' and 104b' of thermo-conductive segments 104a and 104b, respectively, inducing thermo-conductive segment 104b opening up from thermo-conductive segment 104a, allowing the actuator-induced piercing device 110 protrusion through the opening gap, denoted as 30, formed between the thermo-conductive segments, as shown in Fig. 6b.

In their opened position, showed in Fig. 6b, side 144a' (not shown) of thermo-conductive plate 144a is facing side 144b' (not shown) of thermo-conducting plate 144b but the plates are not contacting each other. According to some embodiments, thermo-conductive segment 104b, including thermo-conductive plate 144b, TEC elements 130b' and 130b", insulator 134b, and panel 132b (shown in Fig. 3) are configured to depart from thermo-conductive segment 104a by moving in a third-class lever mode, wherein the fulcrum may be represented by edge 132b' of panel 132 of thermo-conductive segment 104b. Thermo-conductive segment 104a, is fixedly connected to piercing element housing/heat absorber 102, such that its motion is restricted and only thermo-conductive segment 104b is allowed to move upon contacting of circumferential element 312 of piercing element 110 with edge 104a' of thermo-conductive segment 104a and edge 104b' of thermo-conductive segment 104b.

According to other embodiments, both thermo-conductive segment 104b, including thermo-conductive plate 144b, TEC elements 130b' and 130b", insulator 134b, and panel 132b and thermo-conductive segment 104a, including thermo-conductive plate 144a, TEC elements 130a' and 130a", insulator 134a, and panel 132a (shown in Fig. 3) may be configured to move in a third-class level mode, upon extracting of piercing element 110, departing from one another.

According to alternative embodiments, thermo-conductive segment 104a, including thermo-conductive plate 144a, TEC elements 130a' and 130a", insulator 134a, and panel 132a may be configured to depart from thermo-conductive segment 104b by moving in a third-class lever mode, wherein thermo-conductive segment 104b is fixedly connected to piercing element housing/heat absorber 102, such that its motion is restricted and only thermo-conductive segment 104a is allowed to move upon contacting of circumferential element 312 of piercing element 110 with edge 104a' of thermo-conductive segment 104a and edge 104b' of thermo-conductive segment 104b (not shown).

Opening of thermo-conductive segment 104b from thermo-conductive segment 104a, due to the motor 106 functioning, produces opening gap 30 between the thermo-conductive segments, sufficient to facilitate protrusion of piercing element 110 through thermo-conductive segments 104a and 104b and provide extension of needle 302 beyond thermo-conductive plates edges 146a and 146b, contacting skin target area, such that needle 302 can contact skin and further pierce it to obtain a blood sample.

In the opened position of thermo-conductive segments 104a and 104b and the fully-extended state of piercing element 110 (wherein needle 302 contacts skin target area and pierces the skin), edge 146a of thermo-conductive plate 144a continues to contact skin target area, and cool or heat the skin surface. Edge 146a of thermo-conductive plate 144a further continues to exert pressure on the skin in the vicinity of the piercing point, assuring blocking blood flow out of the piercing point, after needle 302 is ejected from the piercing point and piercing element 110 is being retracted back into piercing element housing/heat absorber 102.

Upon reaching the predetermined incision depth, motor 106 radial motion is reversed, allowing actuator 108 to induce piercing element 110 backwards motion, until piercing element 110 is retracted back into piercing element housing/heat absorber 102 to its original position. Rejoining of thermo-conductive segments 104a and 104b is enabled when circumferential element 312 of piercing element 110 detaches from edge 104a' of thermo-conductive segment 104a and from edge 104b' of thermo-conductive segment 104b, and no pressure is being exerted on the edges 104a' and 104b' of the thermo-conductive segments. The contact between the skin target area and the distal end 146a of thermo-conductive plate 144a is reestablished, allowing cooling and or heating and exerting pressure on the skin target area by both thermo-conductive segments 104a and 104b.

According to some embodiments, the present invention blood sampling device 100 (shown in Fig. 1) and/or blood sampling system 200 (shown in Fig. 2) may be used for fingertip piercing including a heating step, at temperatures not higher than 20-35 degrees C, before and/or during and/or after skin pricking, and wherein the blood test strips may measure cholesterol, or other blood parameters/values, which requires larger volumes of blood samples comparing to regular fingertips blood sampling.

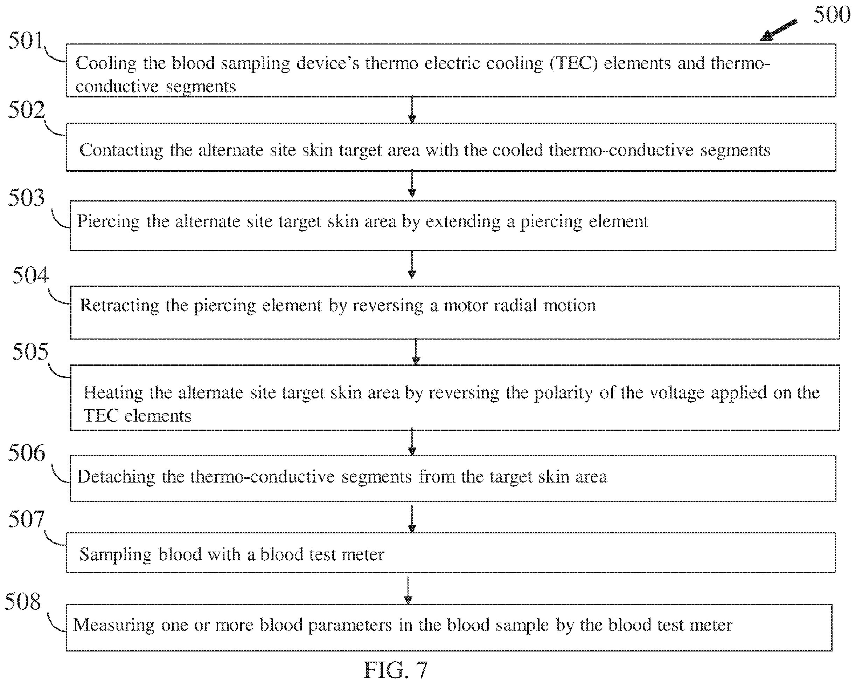

Reference is made to **Figure 7****,** which schematically illustrates a flow chart 500 of an exemplary mode of general operation of blood sampling system 200 (shown in Fig. 2) for measuring blood glucose.
[STEP 501] Cooling the blood sampling device's TEC elements and thermo-conductive segments. The blood sampling device microcontroller activates the cooling of TEC elements 130a', 130a", 130b' and 130b (shown in Fig. 3) and the TEC elements cool thermo-conductive plates 144a and 144b (shown in Fig. 3) of thermo-conductive segment 104a and 104b (shown in Fig. 3). Upon reaching the predetermined temperature and/or cooling time of thermo-conductive plates 144a and 144b, the temperature sensor is configured to transmit an indication to the microcontroller, indicating that system 200 is ready for use.
[STEP 502] Contacting an alternate site target skin area with the cooled thermo-conductive segments. Upon receiving a signal from system 200, the user may attach system 200 to the skin target area, where thermo-conductive segments 104a and 104b, and more specifically the distal end edges 146a and 146b (shown in Fig. 3) of thermo-conductive plates 144a and 144b, respectively, are in direct contact with the skin target area. If within predetermined time a skin contact is not detected by the device, the microcontroller is configured to stop TEC operation and to shut-down system 200. If within predetermined time the device detects a skin, thermo-conductive segments 104a and 104b cool the skin, thereby shrinking blood vessels and capillaries in the skin target area, essentially blocking blood flow into the constricted blood vessels and creating high blood pressure in the skin target area cappilaries. In order to improve the efficacy of the blockage, thermo-conductive segment 104a and 104b may be pressed by the user against the skin surface, before, during and after skin piercing.

It should be noted that the cooling step is performed for several seconds, typically 5 to 7 seconds, in order to increase as much as possible the internal blood pressure developed inside the capillaries located in the vicinity of the piercing site and to cryo-local-anestheized the piercing site.

A temperature sensor may be configured to detect a momentary temperature increase of the TEC elements due to contacting a skin target area having a higher temperature than the thermo-conductive segments. Upon detecting the momentary temperature increase, a timer may be activated by the microcontroller. Upon reaching a predetermined time, sufficient for cooling the skin target area, the timer may be configured to transmit a signal to the microcontroller indicating that piercing may be performed.

[STEP 503] Piercing the alternate site target skin area by extending a piercing element. Upon receiving a signal that the device is ready and piercing may be performed, the microcontroller is configured to activate motor 106 (shown in Fig. 6b) pushing piercing element 110 (shown in Fig. 4a) forward. More specifically, motor 106 activates a shaft, assembled to a screw, wherein the screw that is assembled to a nut is configured to move the nut (and accordingly the piercing element) forward. Piercing element 110 may be configured to induce pressure on thermo-conductive segments 104a and 104b (shown in Fig. 5a), including thermo-conductive plates 144a and 144b (shown in Fig. 6a), respectively, opening up a gap 30 (shown in Fig. 6b) between thermo-conductive segment 104b and thermo-conductive segment 104a, and transfer them into their opened position. Piercing element 110 is configured to protrude through the gap opened between thermo-conductive segments 104a and 104b and needle 302 (shown in Fig. 4a) is configured to extend beyond edges 146a and 146b of thermo-conductive plates 144a and 144b, and to pierce the skin target area. The opening of thermo-conductive segment 104b from thermo-conductive segment 104a may be assisted, for example, by a spring or by a plurality of springs, such as two, three or more springs. Piercing of the skin by needle 302 proceeds to a predetermined depth and/or at a predetermined velocity. The protrusion of piercing element 110 and the subsequent piercing of the skin by needle 302 is terminated in response to a signal received from the microcontroller.

[STEP 504] Retracting the piercing element by reversing motor 106 radial motion. Puncture depth is controlled by the microcontroller. Upon reaching the predetermined puncture depth, the microcontroller is configured to transmit a signal to motor 106 and to reverse the direction of the motor radial motion thereof. Reversing of the rotation direction retracts piercing element 110 and allow insertion of piercing element 110 back into piercing element housing/heat absorber 102 (shown in Fig. 1). The retraction of piercing element 110 allows rejoining of thermo-conductive segment 104a with thermo-conductive segment 104b into their closed position and the contact between the skin target area and thermo-conductive segment 104b is therefore reestablished.

[STEP 505] Heating the alternate site target skin area by reversing the polarity of the voltage applied on the TEC elements. Upon retracting piercing element 110, the polarity of voltage applied to TEC elements 130a', 130a", 130b' and 130b" is reversed in order to heat thermo-conductive plates 144a and 144b of segments 104a and 104b, respectively. The heating is performed for a predetermined period of time. The temperature of the skin target area contacting the thermo-conductive segments increases, releasing the blockage of the constricted blood vessels under the thermo-conductive segments. Heating the skin expands the constricted capillaries to their normal diameter, or to a larger diameter, where the accumulated pressure within capillaries in the vicinity of the thermo-conductive segments skin contact area, boost the blood flow velocity in the capillaries to the incision.

Edges 146a and 146b (shown in Fig. 3) of thermo-conductive plates 144a and 144b exert pressure on the skin target area, inhibiting blood flow from the piercing point. Upon reaching the predetermined heating time and/or temperature, the microcontroller may be configured to transmit an indication to the user, indicating that thermo-conductive segments 104a and 104b may be removed from the skin target area. The indication may be maintained until the user removes the thermal-conductive segments 104a and 104b from the skin, and the piercing procedure is completed.

[STEP 506] Detaching the thermo-conductive segments from the target skin area. Upon receiving the signal from the microcontroller of system 200, thermo-conductive-segments 104a and 104b may be detached/removed from the skin surface, which is followed by a boost of blood flow ejection from the punctured site.

[STEP 507] Sampling blood with a blood test meter 202 (shown in Fig. 2). The ejected blood may be sampled by test strip 210a extending from BGM 202 at the puncture site (shown in Fig. 2).

[STEP 508] Measuring one or more blood parameters in the blood sample. Upon sampling the ejected blood with the blood test meter, BGM 202 is activated to measure glucose concentration, for example, sampled by test strip 210a. The one or more blood parameters may be glucose, hemoglobin, ketones, cholesterol, blood coagulants or any combination thereof.

Cooling step 502 may be configured to alleviate pain associated with piercing. Alternate sites skin allows using a cooling step as an anesthetic step by using a closed loop process based on cooling during a predetermined time required to achieve a pain-free skin piercing as described herein. Using cryo-local-anesthesia, by the device and/or the system of the present invention, is enabled by replacing fingertips SBGM by alternate site SBGM, since alternate site skin contains a lower density of thermal fibers (compared to the density of thermal fibers in fingertip's skin), such that the alternate sites skin is less susceptible to cooling burns. Thus, an important advantage of the device and the method of the present invention is enabling using cryo-local-anesthesia providing painless SBGM testing.

Heating step 505 enhances healing of the piercing site incision. Heat therapy increases blood flow to the incision providing proteins, nutrients, and oxygen healing faster to the skin injury.

The combination of cooling step 502 and heating step 505 reduces waiting time for obtaining a blood sample, where the cooling step increases the cappilaries blood pressure and the heating step boost the blood flow decreasing the time required for obtaining a sufficient volume blood sample.

Heating step 505 may be additionally performed after step 506. In these embodiments, the additional heating step 505, allows reducing the waiting time for reusing the device for the next blood test to about 2 minutes, reducing the passive cooling time that may take about 10 to 15 minutes. The heating is performed automatically after removing the device from the skin, triggered by a signal received from the microcontroller to reverse TEC elements 130a', 130a", 130b' and 130b" polarity.

Thermal heat absorber 102 faces sides 133b' and 133b" of respective TEC elements 130b' and 130b" (shown in Fig. 3) are cooled during heating step 505. After the first round of cooling of the heat absorber, the temperature sensor is configured to indicate to the microcontroller if the predetermined temperature of heat absorber 102 is achieved. If the temperature is still above the predetermined temperature, the microcontroller is configured to reactivate TEC elements 130a', 130a", 130b' and 130b", such that sides 133b' and 133b" of respective TEC elements 130b' and 130b", facing heat absorber 102 are cooled further, thereby reducing the temperature of heat absorber 102.

Heating step 505 is repeated until heat absorber 102 and heat insulator 204 (shown in Fig. 2) temperature is reduced to the predetermined value. The closed loop cooling process of the heat absorber by TEC elements 130a', 130a", 130b' and 130b", specifically by sides 133b' and 133b" of respective TEC elements 130b' and 130b, implementing heating step 505 allows reusing the device for the next blood test after a shorter time of about 2 minutes.

Step 505, applied before and/or after step 506 is configured to reduce the temperature in the vicinity of BGM 202, where the temperature of BGM 202 should not increase beyond about 39°C to ensure glucose measurement accuracy.

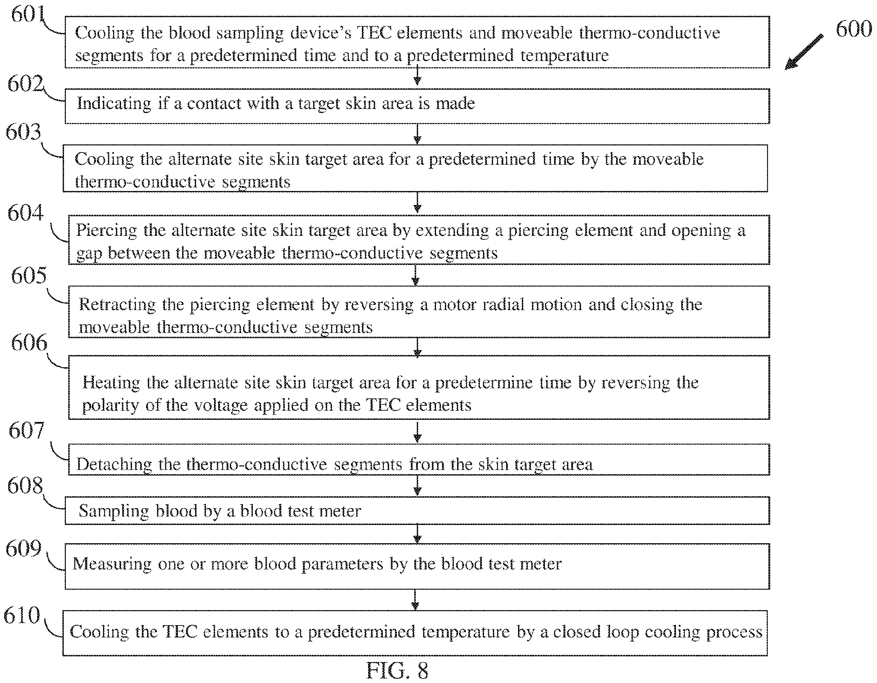

Reference is made to **Figure 8** which schematically illustrates a flow chart 600 of an additional exemplary mode of operation of blood sampling system 200 for measuring blood glucose.
[STEP 601] Cooling the blood sampling device's TEC elements and moveable thermo-conductive segments for a predetermined time and to a predetermined temperature. Step 601 may include pressing an operating button that activates the TEC elements cooling of the movable thermo-conductive segments to a predetermined temperature for a predetermined time.
[STEP 602] Indicating if a contact with a skin target area is made. Step 602 may include providing to the user the following indications:
   If within a predetermined time the device does not detect the skin, the TEC elements cooling may be stoped.
   If within the predetermined time the device detects the skin, the cooling process may continue.
[STEP 603] Cooling the alternate site skin target area for a predetermined time by the moveable thermo-conductive segments.
[STEP 604] Piercing the alternate site skin target area by extending a piercing element and opening a gap 30 (shown in Fig. 6b) between the moveable thermo-conductive segments. Step 604 may include activating motor 106 at a predetermined speed in order to extend the piercing element. Upon extension of the piercing element, the movable thermo-conductive segments open up, allowing a lancet to pierce the skin target area.
[STEP 605] Retracting the piercing element by reversing the motor radial motion and closing the moveable thermo-conductive segments. Step 605 may include changing the direction of motor 106 (shown in Fig. 1) radial motion to retract the piercing element into the piercing element housing. Upon retaction of the piercing element, the thermo-conductive segments may be closed automatically, by a spring for example.
[STEP 606] Heating the alternate site skin target area for a predetermine time by reversing the polarity of the voltage applied on the TEC elements. Step 606 may include reversing the polarity of the voltage applied on the TEC elements, thereby heating the movable thermo-conductive segments. The thermo-conductive segments being in contact with the skin surface heat the piercing site for a predetermined time.
[STEP 607] Detaching the thermo-conductive segments from the skin target area.
[STEP 608] Sampling blood by a blood test meter. Step 608 may include contacting the blood sample accumulated at the piercing site by a blood test meter, such as FIG. 2, BGM 202 that may include test strips.
[STEP 609] Measuring one or more blood parameters by the blood test meter. Step 609 may include activating a blood test meter, for example BGM 202, to measure glucose level sampled by a test strip. The one or more blood parameters may be glucose, cholesterol, hemoglobin, ketones, blood coagulants or combinations thereof.
[STEP 610] Cooling the TEC elements to a predetermined temperature by a closed loop cooling process. Step 610 may include reactivating the TEC elements to cool heat absorber 204 (shown in Fig. 2) until its temperature is reduced to a predetermined temperature, regulated by a closed loop process, wherein the hot end of the TEC elements is at a lower temperature than the cold end in contact with the heat absorber, thus cooling heat absorber 204. After heat absorber 204 temperature decreases to a predetermined temperature, detected by a temperature sensing means, the closed loop process is configured to deactivate the TEC elements and to shut down. The blood sampling device 100 is ready for the next blood test.

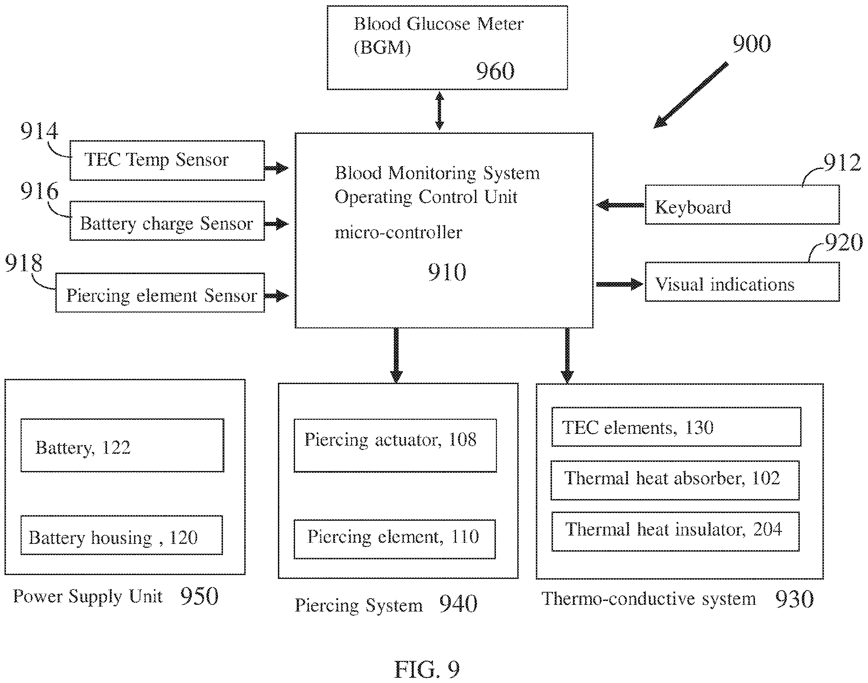

Reference is made to **Figure 9****,** which schematically illustrates a block diagram 900 of blood sampling system 200 (shown in Fig. 2) for measuring blood glucose, in accordance with some embodiments of the invention.

The electronic components of blood sampling system 200 may be regulated by an operating control unit (OCU) programmed microcontroller 910. Sensors, such as TEC temperature sensor 914 and piercing element sensor 918 may be configured to transmit an indication of temperature and piercing depth, respectively, to microcontroller 910. Microcontroller 910 may be configured to receive an input from a keyboard input device 912, which allows the user to input and modify a plurality of device parameters, to read device parameters, to read stored results, calculated statisitics and the like. Microcontroller 910 may be configured to provide a plurality of visual indications to users 920. Microcontroller 910 may be configured to receive indications and to control the operations of the blood sampling system 200 that may include a thermo-conductive system 930, piercing system 940, power supply unit 950 and blood glucose meter 960.

For example, microcontroller 910 may be configured to send an indication to motor 106 (shown in Fig. 1) which is a part of piercing system 940, wherein the motor may be configured to reverse its rotational direction, upon receiving an indication from microcontroller 910. Microcontroller 910 may be configured to send an indication to TEC elements 130 of the thermo-conductive system 930, wherein TEC elements may be configured to reverse their polarity (from cooling to heating polarity), upon receiving the indication from microcontroller 910. Thermo-conductive system 930 may include TEC elements 130, thermal heat absorber 102 and thermal heat insulator 204.

Microcontroller 910 may be configured to send visual indications to users 920, indicating, for example, that blood sampling system 200 is ready for use. Microcontroller 910 may be configured to receive power supply signals, regarding the battery status for example, and may provide to users visual indications accordingly 920. Microcontroller 910 may be configured to receive indications and readings from blood glucose meter 960 and may be configured to instruct blood glucose meter 960 to perform glucose measurement. Power supply unit 950 may include battery 122 and batery housing 120. Power supply unit 950 may include a plug-in cable for external power supply (not shown).

While a number of exemplary aspects and embodiments have been discussed above, those of skill in the art will recognize certain modifications, permutations, additions and subcombinations thereof.

In the description and claims of the application, each of the words "comprise" "include" and "have", and forms thereof, are not necessarily limited to members in a list with which the words may be associated.

## Claims

1. A blood sampling device (100) for alternate site blood testing, the device comprising:
a thermo-conductive member (104a; 104b) configured to cool and heat a skin area;
a piercing system, comprising:
a piercing element (110) configured to pierce the skin area; and
a piercing mechanism for extending and retracting said skin piercing element (110); and an operating control unit (112) configured to control the temperature of said thermo-conductive member (104a; 104b) and to trigger the extension of said piercing element (110); wherein controlling the temperature of said thermo-conductive member (104a; 104b) comprises cooling said thermo-conductive member (104a; 104b) to a predetermined temperature within the range of -10°C -+10°C for a predetermined time, capable of blocking blood flow in capillary vessels at the skin area and of accumulating a pressure within the blocked capillary vessels, trigger the extension of said piercing element (110) upon receiving an indication that said predetermined temperature has been reached, and heating said thermo-conductive member (104a; 104b) to a predetermined temperature within the range of +20°C-+50°C for a predetermined time, capable of removing said blockage and of releasing the accumulated pressure in the capillary vessels and causing a boost of blood flow therein.

2. The device according to claim 1, wherein said thermo-conductive member (104a; 104b) is configured to directly contact said skin area, including a skin piercing point.

3. The device according to claim 2, wherein said thermo-conductive member (104a; 104b) is configured to directly contact said skin area, including said skin piercing point, during cooling and heating of said skin area.

4. The device according to claim 1, wherein said thermo-conductive member (104a; 104b) is further configured to exert pressure on said skin area.

5. The device according to claim 1, further comprising at least one thermo-electric cooling (TEC) element (130) in thermal contact with the thermo-conductive member (104a; 104b), wherein the TEC element (130) is configured to cool and/or heat said thermo-conductive member (104a; 104b).

6. The device according to claim 5, wherein said at least one TEC element (130) is configured to switch between cooling and heating of said thermo-conductive member (104a; 104b) upon reversing the polarity of the voltage applied on said at least one TEC element (130).

7. The device according to claim 1, wherein said thermo-conductive member (104a; 104b) is configured to provide an opening through which a protrusion of said piercing element (110) is facilitated.

8. The device according to claim 7, wherein said thermo-conductive member (104a; 104b) comprises at least two longitudinally aligned segments (104a; 104b).

9. The device according to claim 8, wherein distal edges of said at least two longitudinally aligned segments (104a; 104b) are configured to abut during heating and/or cooling of said skin area including the point of the skin area to be pierced.

10. The device according to claim 9, wherein the control unit (112) is configured to induce an opening between distal edges of the at least two segments (104a; 104b), through which said piercing element (110) can be extended, upon receiving the indication that said predetermined temperature has been reached.

11. The device according to claim 1, wherein the thermo-conductive member (104a; 104b) comprises a heat absorber, configured to accumulate excess heat.

12. A blood sampling system comprising the device (100) of claim 1 and a blood test meter configured to monitor blood glucose level.

13. The system according to claim 12, further comprising a heat absorber or an insulating partition between said blood sampling device (100) and said blood test meter.

## Patentansprüche

1. Blutentnahmevorrichtung (100) für Blutprüfungen an alternativen Stellen, wobei die Vorrichtung Folgendes umfasst:
ein wärmeleitendes Glied (104a; 104b), das zum Kühlen und Erwärmen eines Hautbereichs ausgestaltet ist,
ein Einstechsystem, das Folgendes umfasst:
ein Einstechelement (110), das zum Einstechen in den Hautbereich ausgestaltet ist, und
einen Einstechmechanismus zum Ausfahren und Zurückziehen des Hauteinstechelements (110),
und eine Betriebssteuereinheit (112), die dazu ausgestaltet ist, die Temperatur des wärmeleitenden Glieds (104a; 104b) zu steuern und das Ausfahren des Einstechelements (110) auszulösen, wobei das Steuern der Temperatur des wärmeleitenden Glieds (104a; 104b) das Kühlen des wärmeleitenden Glieds (104a; 104b) auf eine vorbestimmte Temperatur in dem Bereich von -10°C - +10°C für eine vorbestimmte Zeit umfasst, wodurch die Durchblutung in Kapillargefäßen an dem Hautbereich blockiert und Druck in den blockierten Kapillargefäßen gestaut wird, das Ausfahren des Einstechelements (110) bei Erhalt einer Anzeige, dass die vorbestimmte Temperatur erreicht worden ist, auszulösen, sowie das Erhitzen des wärmeleitenden Glieds (104a; 104b) auf eine vorbestimmte Temperatur in dem Bereich von +20°C - +50°C für eine vorbestimmte Zeit, wodurch die Blockierung aufgehoben und der gestaute Druck in den Kapillargefäßen abgelassen und eine vermehrte Durchblutung darin veranlasst werden kann.

2. Vorrichtung nach Anspruch 1, wobei das wärmeleitende Glied (104a; 104b) dazu ausgestaltet ist, den Hautbereich, einschließlich eines Hauteinstechpunkts, direkt zu kontaktieren.

3. Vorrichtung nach Anspruch 2, wobei das wärmeleitende Glied (104a; 104b) dazu ausgestaltet ist, den Hautbereich, einschließlich des Hauteinstechpunkts, während des Kühlens und Erwärmens des Hautbereichs direkt zu kontaktieren.

4. Vorrichtung nach Anspruch 1, wobei das wärmeleitende Glied (104a; 104b) ferner dazu ausgestaltet ist, Druck auf den Hautbereich auszuüben.

5. Vorrichtung nach Anspruch 1, ferner umfassend mindestens ein thermoelektrisches Kühlelement (TEC) (130) in thermischem Kontakt mit dem wärmeleitenden Glied (104a; 104b), wobei das TEC-Element (130) dazu ausgestaltet ist, das wärmeleitende Glied (104a; 104b) zu kühlen und/oder zu erwärmen.

6. Vorrichtung nach Anspruch 5, wobei das mindestens eine TEC-Element (130) dazu ausgestaltet ist, beim Umkehren der Polarität der Spannung, mit der das mindestens eine TEC-Element (130) beaufschlagt ist, zwischen Kühlen und Erwärmen des wärmeleitenden Glieds (104a; 104b) zu wechseln.

7. Vorrichtung nach Anspruch 1, wobei das wärmeleitende Glied (104a; 104b) dazu ausgestaltet ist, eine Öffnung bereitzustellen, durch die ein Vorspringen des Einstechelements (110) ermöglicht wird.

8. Vorrichtung nach Anspruch 7, wobei das wärmeleitende Glied (104a; 104b) mindestens zwei in Längsrichtung ausgerichtete Segmente (104a; 104b) umfasst.

9. Vorrichtung nach Anspruch 8, wobei distale Ränder der mindestens zwei in Längsrichtung ausgerichteten Segmente (104a; 104b) dazu ausgestaltet sind, während des Erwärmens/Kühlens des Hautbereichs, einschließlich des Punkts des Hautbereichs, in den einzustechen ist, aneinander anzuliegen.

10. Vorrichtung nach Anspruch 9, wobei die Steuereinheit (112) dazu ausgestaltet ist, nach Erhalt der Anzeige, dass die vorbestimmte Temperatur erreicht worden ist, eine Öffnung zwischen distalen Rändern der mindestens zwei Segmente (104a; 104b) zu induzieren, durch die das Einstechelement (110) ausgefahren werden kann.

11. Vorrichtung nach Anspruch 1, wobei das wärmeleitende Glied (104a; 104b) einen Wärmeabsorber umfasst, der zur Speicherung von überschüssiger Wärme ausgestaltet ist.

12. Blutentnahmesystem, umfassend die Vorrichtung (100) nach Anspruch 1 und ein Bluttestmessgerät, das zur Überwachung des Blutzuckerspiegels ausgestaltet ist.

13. System nach Anspruch 12, ferner umfassend einen Wärmeabsorber oder eine Isoliertrennwand zwischen der Blutentnahmevorrichtung (100) und dem Bluttestmessgerät.

## Revendications

1. Dispositif (100) de prise de sang pour un test sanguin en site de service alternatif, le dispositif comprenant :
un élément thermoconducteur (104a ; 104b) configuré pour refroidir et chauffer une zone cutanée ;
un système de perçage, comprenant :
un élément (110) de perçage configuré pour percer la zone cutanée ;
un mécanisme de perçage pour sortir et rentrer ledit élément (110) de perçage cutané ; et
une unité (112) de commande de fonctionnement configurée pour commander la température dudit élément thermoconducteur (104a ; 104b) et pour
déclencher la sortie dudit élément (110) de perçage, dans lequel la commande de la température dudit élément thermoconducteur (104a ; 104b) comprend les opérations consistant à refroidir ledit élément thermoconducteur (104a ; 104b) à une température prédéterminée dans la plage de -10°C à +10°C pendant une durée prédéterminée, capable de bloquer le flux sanguin dans les vaisseaux capillaires au niveau de la zone cutanée et d'accumuler une pression à l'intérieur des vaisseaux capillaires bloqués, déclencher la sortie dudit élément (110) de perçage lors de la réception d'une indication que ladite température prédéterminée a été atteinte, et chauffer ledit élément thermoconducteur (104a ; 104b) à une température prédéterminée dans la plage de +20°C à +50°C pendant une durée prédéterminée, capable d'éliminer ledit blocage et de libérer la pression accumulée dans les vaisseaux capillaires et d'y provoquer une augmentation du flux sanguin.

2. Dispositif selon la revendication 1, dans lequel ledit élément thermoconducteur (104a ; 104b) est configuré pour entrer directement en contact avec ladite zone cutanée, comprenant un point de perçage cutané.

3. Dispositif selon la revendication 2, dans lequel ledit élément thermoconducteur (104a ; 104b) est configuré pour entrer directement en contact avec ladite zone cutanée, comprenant ledit point de perçage cutané, pendant le refroidissement et le chauffage de ladite zone cutanée.

4. Dispositif selon la revendication 1, dans lequel ledit élément thermoconducteur (104a ; 104b) est en outre configuré pour exercer une pression sur ladite zone cutanée.

5. Dispositif selon la revendication 1, comprenant en outre au moins un élément (130) de refroidissement thermoélectrique (ERT) en contact thermique avec l'élément thermoconducteur (104a ; 104b),
dans lequel l'élément ERT (130) est configuré pour refroidir et/ou chauffer ledit élément thermoconducteur (104a ; 104b).

6. Dispositif selon la revendication 5, dans lequel ledit élément ERT (130) est configuré pour passer du refroidissement au chauffage dudit élément thermoconducteur (104a ; 104b) lorsque l'on inverse la polarité de la tension appliquée audit élément ERT (130).

7. Dispositif selon la revendication 1, dans lequel ledit élément thermoconducteur (104a ; 104b) est configuré pour offrir une ouverture à travers laquelle l'avancée dudit élément (110) de perçage est facilitée.

8. Dispositif selon la revendication 7, dans lequel ledit élément thermoconducteur (104a ; 104b) comprend au moins deux segments (104a ; 104b) alignés longitudinalement.

9. Dispositif selon la revendication 8, dans lequel les bords distaux desdits segments (104a ; 104b) alignés longitudinalement sont configurés pour venir en butée pendant le chauffage et/ou le refroidissement de ladite zone cutanée comprenant le point de la zone cutanée à percer.

10. Dispositif selon la revendication 9, dans lequel l'unité (112) de commande est configurée pour entraîner une ouverture entre les bords distaux desdits segments (104a ; 104b), à travers laquelle on peut faire sortir ledit élément (110) de perçage lorsque l'on reçoit l'indication que ladite température prédéterminée a été atteinte.

11. Dispositif selon la revendication 1, dans lequel l'élément thermoconducteur (104a ; 104b) comprend un absorbeur de chaleur, configuré pour accumuler la chaleur en excédent.

12. Système de prise du sang comprenant le dispositif (100) selon la revendication 1 et un appareil de mesure d'analyse sanguine configuré pour surveiller la glycémie.

13. Système selon la revendication 12, comprenant en outre un absorbeur de chaleur ou une cloison isolante entre ledit dispositif (100) de prise de sang et ledit appareil de mesure d'analyse sanguine.
